# EUROPEAN PATENT SPECIFICATION

(11) **EP 1 085 090 B1**
(45) Date of publication and mention of the grant of the patent: **14.12.2011**
(21) Application number: 99922568.3
(22) Date of filing: 28.05.1999
(51) Int. Cl.: C12N 15/12, C07K 14/47, C12Q 1/68, C12P 21/02, C07K 16/18, G01N 33/53, G01N 33/577, A61K 38/17

(54) **IgA NEPHROPATHY-ASSOCIATED DNA**
MIT IgA-NEPHROPATHIE ASSOZIIERTE DNS
ADN ASSOCIE A UNE GLOMERULONEPHRITE A DEPOTS MESANGIAUX D'IgA

(30) Priority: 02.06.1998 JP 15260398
(43) Date of publication of application: 21.03.2001
(60) Divisional application: 08006344.9
(73) Proprietor: Nihon University, Tokyo 102-8275 (JP)
(72) Inventor: ISHIWATA, Tetsuyoshi, Chiyoda-ku Tokyo 100-8185 (JP); SAKURADA, Mikiko, Machida-shi Tokyo 194-8533 (JP); KAWABATA, Ayako, Machida-shi, Tokyo 194-8533 (JP); NAKAGAWA, Satoshi, Machida-shi, Tokyo 194-8533 (JP); NISHI, Tatsunari, Chiyoda-ku Tokyo 194-8185 (JP); KUGA, Tetsuro, Hofu-shi Yamaguchi 747-8522 (JP); SAWADA, Shigemasa, Musashino-shi Tokyo 180-0004 (JP); TAKEI, Masami, Sayama-shi Saitama 350-1301 (JP); SHIBATA, Kenji, Machida-shi, Tokyo 194-8533 (JP); FURUYA, Akiko, Machida-shi Tokyo 194-8533 (JP)
(74) Representative: Vossius & Partner
(86) International application number: PCT/JP1999/002855
(87) International publication number: WO 1999/063085

(56) References cited:
- EP-A- 0 915 156
- WO-A-99/26980
- WO-A1-98/24899
- DATABASE EMBL [Online] Accession Nr. AA772278, 31 January 1998 (1998-01-31) "ai42d03.s1 Soares_parathyroid_tumor_NbHPA Homo sapiens cDNA clone 1359653 3', mrNA sequence" XP002175919
- DATABASE EMBL [Online] Accession Nr. AA634469, 31 October 1997 (1997-10-31) HIILIER L. ET AL.: "zu76a06.s1 Soares_testis_NHT Homo sapiens cdNA clone IMAGE:743890 3', mRNA sequence." XP002175920
- DATABASE EMBL [Online] Accession Nr. AA381126, 18 April 1997 (1997-04-18) ADAMS M.D. ET AL.: "EST94221 Activated T-cells I Homo sapiens cDNA 5' end similar to C. elegans hypotetical protein, cosmid C40H1." XP002175921
- DATABASE EMBL [Online] Accession Number AL023657, 22 May 1998 (1998-05-22) COFFEY AJ AND BROOKSBANK RA: "Homo sapiens SH2D1A cDNA, formerly known as DSHP." XP002190247 & COFFEY A J ET AL: "HOST RESPONSE TO EBV INFECTION IN X-LINKED LYMPHOPROLIFERATIVE DISEASE RESULTS FROM MUTATIONS IN AN SH2-DOMAIN ENCODING GENE" NATURE GENETICS, NEW YORK, NY, US, vol. 20, no. 2, October 1998 (1998-10), pages 129-135, XP002929123 ISSN: 1061-4036
- SAYOS J ET AL: "THE X-LINKED LYMPHOPROLIFERATIVE-DISEASE GENE PRODUCT SAP REGULATES SIGNALS INDUCED THROUGH THE CO-RECEPTOR SLAM" NATURE, MACMILLAN JOURNALS LTD. LONDON, GB, vol. 395, no. 6701, October 1998 (1998-10), pages 462-469, XP002929122 ISSN: 0028-0836
- TOYABE S.: "IgA nephropathy-specific expression of the IgA Fc receptors (CD89) on blood phagocytic cells." CLIN. EXP. IMMUNOL., vol. 110, no. 2, November 1997 (1997-11), pages 226-232, XP001017994
- DUQUE N. ET AL.: "Interaction of IgA with Fcalpha receptors of human mesangial cells activates transcription factor nuclear factor-kB and induces expression and synthesis of Monocyte Chemoattractant Protein-1, IL-8, and IFN-inducible Protein-10." J. IMMUNOL, vol. 159, 1 October 1997 (1997-10-01), pages 3474-3482, XP001019020
- ICHINOSE H ET AL: "DETECTION OF CYTOKINE MRNA-EXPRESSING CELLS IN PERIPHERAL BLOOD OF PATIENTS WITH IGA NEPHROPATHY USING NON-RADIOACTIVE IN SITU HYBRIDIZATION" CLINICAL AND EXPERIMENTAL IMMUNOLOGY, OXFORD, GB, vol. 103, no. 1, 1996, pages 125-132, XP002926339 ISSN: 0009-9104
- H. ICHINOSE et al., "Detection of Cytokine mRNA-Expressing Cells in Peripheral Blood of Patients with IgA Nephropathy Using Non-Radioactive in Situ Hybridization", CLINICAL AND EXPERIMENTAL IMMUNOLOGY, (1996), Vol. 103, No. 1, pages 125-132, XP002926339.
- TRACY E. HUNLEY et al., "Angiotensin Converting Enzyme Gene Polymorphism: Potential Silencer Motif and Impact on Progression in IgA Nephropathy", KIDNEY INTERNATIONAL, (1996), Vol. 49, No. 2, pages 571-577, XP002926340.

### SEQUENCE LISTING

<110> KYOWA HAKKO KOGYO CO. , LTD. ,
<120> IgA NEPHROPATHY-ASSOCIATED GENE
<130> 11143
<140>
   <141>
<160> 121
<170> PatentIn Ver. 2.0
<210> 1
   <211> 4276
   <212> DNA
   <213> Homo sapiens
<220>
   <221> CDS
   <222> (53).. (742)
<400> 1
   ttctaccgtt ttttccctgc tttctattcc aggtcagtct tcactgtttc cg atg gaa 58
   gat gga ttc ttg gat gat ggc cgt ggg gat cag cct ctt cat agt ggc 106
   ctg ggt tca cct cac tgc ttc agt cac cag aat ggg gag aga gtg gaa 154
   cga tat tct cga aag gtg ttt gta ggc gga ttg cct cca gac att gat 202
   gaa gat gag atc aca gct agt ttt cgt cgc ttt ggc cct ctg att gtg 250
   gat tgg cct cat aaa gct gag agc aaa tcc tat ttt cct cct aaa ggc 298
   tat gca ttc ctg ctg ttt caa gat gaa agc tct gtg cag gct ctc att 346
   gat gca tgc att gaa gaa gat gga aaa ctc tac ctt tgt gta tca agt 394
   ccc act atc aag gat aag cca gtc cag att cgg cct tgg aat ctc agt 442
   gac agt gac ttt gtg atg gat ggt tca cag cca ctt gac cca cga aaa 490
   act ata ttt gtt ggt ggt gtt cct cga cca tta cga gct gtg gag ctt 538
   gcg atg gta atg gat cgg cta tac gga ggt gtg tgc tac gct ggg att 586
   gat acc gac cct gag cta aaa tac cca aaa gga gct ggg aga gtt gcg 634
   ttc tct aat caa cag agt tac ata gct gct atc agt gcc cgc ttt gtt 682
   cag ctg cag cat gga gag ata gat aaa cgg gta agc ctt ata cta cat 730
   ttt gga aaa ttc tagaaatggt cctctaaatg tgtgattacc aatattagaa 782
   cgggagcatt ttatgacaat aaagtgacag ctgacaattt tgcctataga gttaattatg 842
   gtctataata catgaaataa tgtcctatga atttctttta tctttcagtt ttttgagtag 902
   cctaatcaga acactacaat ttacttgagt taatttaatc ttctctaact tccattcaat 962
   ctcaatccat ccgtccattc attcacttag tttgtaagtc attcaataaa tatttactga 1022
   atcctttgtt ctgtgttata tcaagtatac aaacaggaat gcccttgagg tttcctgccc 1082
   ttttttttgt ttgtttttta atcctgggac atagggaaga cctcagcaag ccctatttct 1142
   caatgaattg tactcacaga tttctttttt tttttttttt tctttttcca cagccgccac 1202
   ctctcaccga tttattcctt agcttggtgt ttcatgtatt caacaaacgt tttagtgctt 1262
   agggcaagaa gttcctgtcc tcatgagttt atttcctagc agatagaact gtatcacttg 1322
   ccagtactac tcagagtgtg gcctgtggac tgacctccag tctgtaaact tagtttgtag 1382
   tgagatagga atttagacca gaatgtgtaa tcaaccacat tactgggcac aatgtttggt 1442
   ccagctggcg attttttttt catagaaagc ctttattgat gagggaagca atatattgat 1502
   ttatattttg gggtcacctt tttatttcat ggcacactgg cactttcatg catgctgact 1562
   ttgatatcca tcactctgag gcattgtgct aaaatagatt gattttatcg tgttgttctc 1622
   aattcaagat gtaaaaatca tcaagtcagt agcagttttt gctttttatg tttcatgtca 1682
   tgtacagtct acttcactgg cagtaaaaaa atttaagata gtggtggtca tcctacaaac 1742
   tgtgaatcta ttaaagagaa aagtatctgt tctattctaa gcatggggga gggacaagat 1802
   tagtatgtta acatgcctac tttgtttgtt tgagatggag tctctctccg tcacccaggc 1862
   tggagtgcag tggtacagtc tcagctcact ccaacctctg cctcccgggt tcaagtgatt 1922
   ctcctgcctt agcctcccga gtaggtggaa ttacaggcat ataccaccat gcccaacaaa 1982
   tgtttgtatt tttagtggag acagggtttc accgtgttgg tcaggccagt ttcaaactcc 2042
   tgacctcaag ggatccacct gcctcacccc ctcaaagtgc tgggattaca ggcatgagcc 2102
   acccaccatg cctggcctac ttggtttttt atgcacacta aaaaatacct acatctcact 2162
   gccttattcc aacataagtt tcagagctgt gggattggtc attagaaatt cagactgaat 2222
   ttgtgttcct ctgcaatgaa atcctttgcc cagtgttcat gtcactctgt agacattatg 2282
   gagcagccta gaggccagaa gcccagtgct ctccttatgc ctgctcttcc tgggcttcgt 2342
   gacactcttc ttctcctttt gtacttttat ttttttagtt aaaaaatttt ttttagaggg 2402
   agggtctcac tctgtcaccc aggctggagc acagaatcac aatcatgact cactgcatgt 2462
   tcttctcctt ttgttcatgg ctaatcttgg tcaggattcc ttgtcagagc tgggtggcac 2522
   cagtgctggt gacagcctgc tgtaagggag tttcagccat gaatctctcc agactaaaaa 2582
   taaccagctc ttttctagct gatgaattaa taaccaggtg actgttaatg cttgaaaggt 2642
   tcacatgaca ggttggccga tagaacgctg gaacaggccc agttttagaa attcacctct 2702
   gacttttaga ctcaggtgaa ccattcttac tgagaaagaa caaagcaggg ttttagactg 2762
   tgaatcctat ggctgcatct tttttttttt tttaacagag ttccaggttt gtgattataa 2822
   cccaacatgt gtacactata aatagaaacc acgagccagg ctttttacga cagctcagaa 2882
   tcttgtgacg cagtagtcag gcatcttcac accgacttga atattgaagt gcagttgtgt 2942
   ggaacttgga tcatcttagt tgattttgtt taaattatga ttccacatat gacaaaaatc 3002
   cagatccact aattaaaatg agggtttatg tctatgaata atctcctgtg ggtttaatct 3062
   cataacattc tagtctaaac agttggcttc acttcatgat gtctgctcaa atcctttttc 3122
   ctttaaagga tgtttattta ataagaaaaa aaatgtaaaa tgatagataa taaaagcctt 3182
   actaggttct taaaagatga actatccata tttcagtaaa tgaataatta gtccttcctc 3242
   tttgggcacc ttggaacaga ttcattcaga tagtgggtgg aaatgtacat gtatggtaag 3302
   cattgctggc ctagtcactg aaaaatgtaa actcttattt ttgattgcag gtggaagtta 3362
   agccatatgt cttggatgat cagctgtgtg atgaatgtca gggggcccgt tgtgggggga 3422
   aatttgctcc atttttctgt gctaatgtta cctgtctgca gtattactgt gaatattgct 3482
   gggctgctat ccattctcgt gctggcaggg aattccacaa gcccctggtg aaggaaggcg 3542
   gtgaccgccc tcggcatatt tcattccgct ggaactaaag gataactgca gtgctcattt 3602
   tcaggcctca gaataagtgc actcttctgt tcattctgac cccttcctca acctcttcac 3662
   gctggcatgt ccttttgtag cagtctgtaa cttaactata gtataatgaa aagaatgacc 3722
   tataatatag gtgttttgta gattcttgtg tcactgcaaa caatatgaac tcctttttcg 3782
   tattgccatc gggttgcatg gaagttttat tctcttgttt tgctggaaac caagaggatc 3842
   caaacttcct gcaacatttt cttagaggag agagagaaat attaaaagag aaatgaaaca 3902
   atagagtatt ttgggttttt aattaaatta ttgttaataa tataacatat aagaatactt 3962
   ttattaaaat aaccatgcaa caataacact atcggtctat ctgacagttt ttcccccagg 4022
   gaagtgcttt tgccttttcc tttctttttt tttttttttc atcttttttg ttctctctct 4082
   tttttccatc cctttttaat ttttttaaca gcaatggagg aagttaacaa tttttaatgg 4142
   aaagagcatg ttagagcaaa caaatgcata agcaagactg agcagcatta taattaattt 4202
   tcagggtttt gaggctgaac ataatttcat tatccctcaa aaagttacca ccacatcaga 4262
   aaaaaaaaaa aaaa                                                    4276
<210> 2
   <211> 2689
   <212> DNA
   <213> Homo sapiens
<220>
   <221> CDS
   <222> (107).. (535)
<400> 2
   gttggaggtt ctggggcgca gaaccgctac tgctgcttcg gtctctcctt gggaaaaaat 60
   aaaatttgaa ccttttggag ctgtgtgcta aatcttcagt gggaca atg ggt tca    115
                                                     Met Gly Ser
                                                     1
   gac aaa aga gtg agt aga aca gag cgt agt gga aga tac ggt tcc atc   163
   Asp Lys Arg Val Ser Arg Thr Glu Arg Ser Gly Arg Tyr Gly Ser Ile
       5                   10                  15
   ata gac agg gat gac cgt gat gag cgt gaa tcc cga agc agg cgg agg   211
   Ile Asp Arg Asp Asp Arg Asp Glu Arg Glu Ser Arg Ser Arg Arg Arg
    20                  25                  30                  35
   gac tca gat tac aaa aga tct agt gat gat cgg agg ggt gat aga tat   259
   Asp Ser Asp Tyr Lys Arg Ser Ser Asp Asp Arg Arg Gly Asp Arg Tyr
                   40                  45                  50
   gat gac tac cga gac tat gac agt cca gag aga gag cgt gaa aga agg   307
   Asp Asp Tyr Arg Asp Tyr Asp Ser Pro Glu Arg Glu Arg Glu Arg Arg
               55                  60                  65
   aac agt gac cga tcc gaa gat ggc tac cat tca gat ggt gac tat ggt   355
   Asn Ser Asp Arg Ser Glu Asp Gly Tyr His Ser Asp Gly Asp Tyr Gly
   70 75 80
   gag cac gac tat agg cat gac atc agt gac gag agg gag agc aag acc 403
   Glu His Asp Tyr Arg His Asp Ile Ser Asp Glu Arg Glu Ser Lys Thr
   85 90 95
   atc atg ctg cgc ggc ctt ccc atc acc atc aca gag agc gat att cga 451
   Ile Met Leu Arg Gly Leu Pro Ile Thr Ile Thr Glu Ser Asp Ile Arg
   100 105 110 115
   gaa atg atg gag tcc ttc gaa ggc cct cag cct gcg gat gtg agg ctg 499
   Glu Met Met Glu Ser Phe Glu Gly Pro Gln Pro Ala Asp Val Arg Leu
   120 125 130
   atg aag agg aaa aca ggt gag agc ttg ctt agt tcc tgatattatt 545
   Met Lys Arg Lys Thr Gly Glu Ser Leu Leu Ser Ser
   135 140
   gttctcttcc ccattcccac ctcagtccct aaagaacatc ctgattcccc cagtcttcaa 605
   gcacatgaat tcagaatgaa aggtttgcca tggctaagga atgtgactct ttgaaaacca 665
   tgttagcatc tgaggaactt ttttaaactt tgttttaggg actttttttt ccttaggtaa 725
   gtaatgattt ataaactcct tttttttttt ttgactatag tcggttgcat ggttacttta 785
   agcgtggaat caaatggagt ggcatttagt tcaggcggct tgttccttgc catggcaaag 845
   tatcaagaag atccccaagt caagtcacat ttgtaaagct gcttcccaat tggctttgtc 905
   acgcagtgtt gaagcagtgg gagagagatt cacctgttat aaaggaactg actaacacaa 965
   gtatcccgtc tatatctgaa tgctgtctct aggtgtaagc cgtggtttcg ccttcgtgga 1025
   gttttatcac ttgcaagatg ctaccagctg gatggaagcc aatcaggttg cttcactcac 1085
   caagtctaga tattcatgaa aatggaacaa gtctgtacaa ttttaaaaaa aggttgaagg 1145
   agtggtttgt tccaaaggag tgactttttt ttaaaaaaaa aagctttgta tatattaaaa 1205
   ttgatgttac tagaataagt acagtaccaa ggacttcatt atagaatttg ttctgccttt 1265
   aaacatggct acctacctgg cagggctttg ttaactactg aatacctgtc tggtaatcac 1325
   taaaacatct taatgtttcc cttttttcta gtttgttata ttcctattat gtccattgag 1385
   agtaagctta gtatatcaaa ctctccattt gacagtgaag agaacatagt gaaagtctgt 1445
   ggcggcattt ttataagtaa ttccttattt ctgcctgaag accacaaagc ctcctggagg 1505
   cgtaactgct cagaccggtc ttcagggaat atttaaggac ttagtggaat ttatgaacaa 1565
   taagtctgat gagattagcc tgggagtggt gtcctgcagc tgtctaatct agttagagtg 1625
   gcattaacat tctaatctcc ttgagaatgc cttttatagt ctgttcaaag caagtcattg 1685
   atggttcttc gaggtagtgt taactgaagt gttcttcagt ttgtcaagat aatgttcagt 1745
   gcttggcact taaataacat tttttgcaag aactccaagg cacattattg aatgccttta 1805
   accaagtgca ttctgggaag tttgcttgac tcattatctt gcttttctgc agcattctgt 1865
   gatttgagtc atccatgaat ccatgaataa aagttacatt ctttgattgg taatattgcc 1925
   atttataaca agactcacta atgagggtat cactttgact gactgatttg ttaaagtttt 1985
   taagcctctc attttcctaa cccagaaatc acagcctgat tttattaaaa gtagagcttc 2045
   attcatttca taccatagat accatcctag taaatccaga acatatacaa ggttcatgtg 2105
   agtctgcttt cttgacatga tagcattgtt tgatgcagtg gatatgtcag aatgactaac 2165
   ctaggagttt aaaactccta agaaactaaa acctgtaaga catttaaaag tctccacaat 2225
   tttaatgtat acaaagctat gttactgtgt aacacattac agttcaaatt cactccagaa 2285
   ataaaaggcc agtaggatta gggactcact ggtagtttgg agtctcccag cacacatccc 2345
   tcctagtggg atgatctatt cacatatctc ccagcttttt tatttttgct tctgtatatc 2405
   acagtgagtg gatggccctt cagctttttc tctcctggcc agacatgcag tcttgccttt 2465
   agatatcgca gagacaaaat tcacagcatg tcttaaatct tccaggattt gcaagaacca 2525
   aattgctcaa cagtatgtat gtttagaggg gttagactcc tttttaaaat ctggatatct 2585
   aaccacctac ttaaatctgt ttgatagtgt caaaccaccc ccacccttga tcctcccacc 2645
   cccaaaaaaa aaaaaaaaaa aaaaaaaaaa aaaaaaaaaa aaaa 2689
<210> 3
   <211> 2981
   <212> DNA
   <213> Homo sapiens
<220>
   <221> CDS
   <222> (1297)..(1608)
<400> 3
   cctctctctc tctttcacag agtcttgctc tgtcgcccag gctggagtgc agtggcacaa 60
   tctcactgca agctccgcct cctgggttca cgccattctc ctgcctcagc ctcccaaata 120
   gctgggacaa caggcacctg ccaccacgcc cggctaattt tttgtatttt tagtagagac 180
   agggtttcac catgttagcc aggatggtct caatctcctg acctcgtgat ccacccgcct 240
   cagcctcccc aaatgctgag attacaggtg tgagccacca cgcccagcca catctttctt 300
   tctttctttt tggtttttgt ttgttgtttg agacagggtc ttgctctgtc gccctggctc 360
   acgtgaacct cccacctcag cctcccaagt agctgagacc acaggtgtga gccaccactc 420
   ctgggtaatg tttgtatttt tttgtagaga tggggtttca ccgtgctgcc cagactgctc 480
   tcaaactcct gggctcaagt gatccacctg ccttgacctc ctaaagtgct ggaattacag 540
   gtgtgagcca ccgtgctcag ccgagtgtct ttcgtatgtt ttctgagcac gtggatttcc 600
   atctctctgc attctctgtt catctcagcc tgtttgttcc attgagataa atgacttttt 660
   cttggtaact tagagtactt tgtgtattta caggttaatc ccttatcaat ttatatcagt 720
   tgctgctatc ttttcttaga ttttcttttt cattttaaaa attacattgt ttcaatgaac 780
   agaattttta agttttaacg tagtccactt tgtccatttt ctttatgacc ggtgcatttt 840
   agggtcttgt ttaagaaatc gttctttatc ctgaggtcat aaagatagtc tactgtattt 900
   tcttttaaga gctgaaaagg tgttttatat ttaatttatt tgggattggc ttttgtgtgg 960
   tggggataag gatcacaatt ttatttcatt ttttttccac ttggttatgc cagtggcccc 1020
   atttccattt tttgaatagt ctttctgtgc agaaaagact tcactagcag agaagtcctg 1080
   agacttaccc ttcaaaaggc cccattcaca aggctagcac ttggcgtgca tctgagaacc 1140
   tggattttgg ggtggttcct ataatgtggt gtatgctgaa cacccacctt tccttctggg 1200
   agtctggaat ttgggtatat gttggacaga ggctgcctaa gtgaccagct tcaacaacag 1260
   ccctgggtgc tgggtcactc atgacccata gacaaa atg cca cac atg ttg tca 1314
   Met Pro His Met Leu Ser
   1 5
   cag ctt att gct gga gga gtt agc aca tcc tgt gtg act gca ctg gga 1362
   Gln Leu Ile Ala Gly Gly Val Ser Thr Ser Cys Val Thr Ala Leu Gly
   10 15 20
   gag gaa act ggt gcc tgg ttc cct gtg tat ttg tcc cac gcc tcc agt 1410
   Glu Glu Thr Gly Ala Trp Phe Pro Val Tyr Leu Ser His Ala Ser Ser
   25 30 35
   ccc ttt gct gat ctc gtt ttt tgt cct ttt gct gag ata aat cac agc 1458
   Pro Phe Ala Asp Leu Val Phe Cys Pro Phe Ala Glu Ile Asn His Ser
   40 45 50
   cag gag tat gac aat atg cgg ggt cct gtg agt cct cct aac aaa cag 1506
   Gln Glu Tyr Asp Asn Met Arg Gly Pro Val Ser Pro Pro Asn Lys Gln
   55 60 65 70
   ttc aat ctg ggg gtg atc ttt ggg atc ccc aac aac tgt cgt ttc ccc 1554
   Phe Asn Leu Gly Val Ile Phe Gly Ile Pro Asn Asn Cys Arg Phe Pro
   75 80 85
   act gat aat aaa ata act gag aag cag cta ttg ggc aat gtt ctg aac 1602
   Thr Asp Asn Lys Ile Thr Glu Lys Gln Leu Leu Gly Asn Val Leu Asn
   90 95 100
   tac cct tgaacattca tgtcttcatc tgaacatcca tctactaccc ctgatttttt 1658
   Tyr Pro
   cagtgcaggg tgcatatcct gtatcaccca ataaatggtc attgatcacc ataggaaagg 1718
   aacagtgaaa gctccacggt ggtttggagg aaggtggcag gcattcagcg gtaacttttt 1778
   tgagcagata gattttatgt ttttgcaatg agtgaaataa attttcccat atctatttaa 1838
   ggttggcaat cattatcttt ttatcatctt ggaacatttg gaattccttt aatatgttta 1898
   gttaggaatt ttctaccttc ctcatcttgt ccgatagttt aaaatcccac agttatttca 1958
   cgggctcctc atacctgcct gtgtgatttc taacatgtca cgctatgcaa ccagttgctt 2018
   ttacttgtag agtgtttctt taggtaatag cttattattg gttatgtgat tacagtgtgt 2078
   taaagacagg tctgtagtta tgtaaaatgc cgtttctctg agtatcatgg tcatttccac 2138
   atatttctct attcatgtat ttgtaagaat atatctattt ttgcagtatt ttatttattt 2198
   attttatttt attttctgaa acggagcctt gttctgtcac ctaggctgga gtgcagtggt 2258
   gtgatctcga ctcactgtga cctccccctc ccaggttcaa gcgattctcc cgcctcatcc 2318
   tcccaagtca ttgggattac agtcacgtgc catgaagccc tgctaatttt ttgtattttt 2378
   agtagagaca ggatttcacc atgttggcga tgctggtttc gaactcctgg tttcgaactc 2438
   ctgacctcaa gtgatccacc tgcctcggcc tcccaaagaa ctgggattat gggcgtgaac 2498
   caccacgcca ggtcagtttt gcagtgtttt aaatactgtt gtctttgaga ggagagaggc 2558
   acgcacatag actatggtga ttaccatcat atactggaaa gtgcaaagtg tagcgcagtt 2618
   aactgtgagc catctcatca aaccctaaca gatgtctcat ttgtccataa aggggcttct 2678
   gtcccataga aattcatgta cccaacctac tcttcaacca tgatttttct ctgatggcct 2738
   gtgtgaacag attaatggtg tccatctaat tccttcccca ctgggggaaa gcaaatcatc 2798
   aggcccattg caaaaactgc tcttggttga gcttcctgcc ttaaatcata cccacagtga 2858
   atggcgtccc tttatcaccg ctaatgactc tgacatctct ctccactcac atgtgagcct 2918
   cctcagctct cgataaacaa gtctgtctcg gttcatttat tctacaaaaa aaaaaaaaaa 2978
   aaa 2981
<210> 4
   <211> 1461
   <212> DNA
   <213> Homo sapiens
<220>
   <221> CDS
   <222> (282)..(680)
<400> 4
   aattcggcac gagcagcttt ctagttggat taggcaacag aatcctttga aaatgtgtgt 60
   gcacagacca ggtggctctc tgggccagtg tactctgaaa gatgtgtgtc ctggcctagc 120
   tggttgagga aaagcagggc aagcctagcc aaatcacaca tcttgaacag ccctcattcg 180
   ttatactaac tttcccacct tctggtgtgt ataggagata aagatggcag acgtgctatt 240
   aggctgccaa tgggagtggg ctctgatatg gtctttcaaa t atg aat cac ccc tgg 296
   Met Asn His Pro Trp
   1 5
   cat gtg tgt ttc ctg ttt aag gtt ctc agg tat tac cca act gca cca 344
   His Val Cys Phe Leu Phe Lys Val Leu Arg Tyr Tyr Pro Thr Ala Pro
   10 15 20
   ata tta aaa tgg aca cat acc gtg tca tgc agt tgg tgc cga agt gtt 392
   Ile Leu Lys Trp Thr His Thr Val Ser Cys Ser Trp Cys Arg Ser Val
   25 30 35
   tta agg gaa gtt gta ggc aat gtg agt tta tca gaa aac ttc acc ata 440
   Leu Arg Glu Val Val Gly Asn Val Ser Leu Ser Glu Asn Phe Thr Ile
   40 45 50
   tca gca ttt tgc cct gag ctt aca cca ttc cca gat caa ggt aca agc 488
   Ser Ala Phe Cys Pro Glu Leu Thr Pro Phe Pro Asp Gln Gly Thr Ser
   55 60 65
   aca atg att tcc ttt ctt gaa aag ttc aac aaa agc aag aga gag aga 536
   Thr Met Ile Ser Phe Leu Glu Lys Phe Asn Lys Ser Lys Arg Glu Arg
   70 75 80 85
   ttg gag ttg atg ctg cat ttt tat tct gtg tta agt ctt gaa cct gct 584
   Leu Glu Leu Met Leu His Phe Tyr Ser Val Leu Ser Leu Glu Pro Ala
   90 95 100
   gtt gct gaa cat tgg tca ggg gaa ttt gag aag tgg aaa gtg ggc ttt 632
   Val Ala Glu His Trp Ser Gly Glu Phe Glu Lys Trp Lys Val Gly Phe
   105 110 115
   ttt cac cct ttg aaa aga gag gat gga ttc ttc acc aga act gac att 680
   Phe His Pro Leu Lys Arg Glu Asp Gly Phe Phe Thr Arg Thr Asp Ile
   120 125 130
   taaaaaaagt cagcgtggca cgttttagta tgtgtggcag atctaaasag acaatatttt 740
   gatctcagga gtgtttattc ttgaaccatt ttcagaactc taagatttga gaaataataa 800
   aatattgacc atccttcaaa gagaaaaaca cagggcgatc tttggcatag cctgtcattt 860
   tgctcacatt tcacttctct ctctccaact tcagagcccc tgctgtggaa caggtgctgt 920
   gctgggtggc aggggaggtc tctggctttt tttttttttg atctccgtct taacatctag 980
   cctactggag gaagtgtatt taatcatcca cttatctgtt aacaattatc tctgagggcc 1040
   cgtcacattc agagaagatt ctaggttctc tacaagtatc ctctcactgt gtacatacta 1100
   aatcaacatc ctgctggatt tcccccagac atctcccttc atcaccattg gagagtatcc 1160
   tctaattgcc agccctattc accatactca tctcatttga tctggagttt tctgagagtg 1220
   accgggggtg ggatggacag gataatttag caagagtgta taagtaaaat ctatataata 1280
   aaagttatct ccctgtgccc cccatgatct attctttatg tagcagtctg aatgagattt 1340
   tcagaaacaa gaaccacttt accttagtct cttcttcttc ttcttcttct tttcttttct 1400
   ttttttttag tattatgggc aacagagcaa gacccagtct caggaaaaaa aaaaaaaaaa 1460
   a 1461
<210> 5
   <211> 3329
   <212> DNA
   <213> Homo sapiens
<220>
   <221> CDS
   <222> (637).. (1035)
<400> 5
   ccaaagtgct gggattatag gcatgagcca ctgcgcccgg ccagaatacc ctatccttaa 60
   acatgaattt aggggagggg aggacacaat tcaatctata acaactatca ctggctgatt 120
   ttggcagagg cctgtggcct ccagtatttt gagggagctg agggccactg atctctccat 180
   atgctctcaa catcatggga ctagtaggat gaaagcaagc ctcagaccag attctacctc 240
   aagcaggcac acaaacattc atgcagcttc tacttggagc ctgatgaagt tcaaattgtt 300
   tgtcctctga ggctctcttt gcatggaaat ttctcccatg acagatgaga aagttctggg 360
   gcagcattca gctttctagt tggattaggc aacagaatcc tttgaaaatg tctgtgcaca 420
   gaccaggtgg ctctctgggc cagtgtactc tgaaagatgt gtgtcctggc ctagctggtt 480
   gaggaaaagc agggcaagcc tagccaaatc acacatcttg aacagccctc attcgttata 540
   ctaactttcc caccctctgg tgtgtatagg agataaagat ggcagacgtg ctattaggct 600
   gccaatggga gtgggctctg atatggtctt tcaaat atg aat cac ccc tgg cat 654
   gtg tgt ttc ctg ttt aag gtt ctc agg tat tac cca act gca cca ata 702
   tta aaa tgg aca cat acc gtg tca tgc agt tgg tgc cga agt gtt tta 750
   agg gaa gtt gta ggc aat gtg agt tta tca gaa aac ttc acc ata tca 798
   gca ttt tgc cct gag ctt aca cca ttc cca gat caa ggt aca agc aca 846
   atg att tcc ttt ctt gaa aag ttc aac aaa agc aag aga gag aga ttg 894
   gag ttg atg ctg cat ttt tat tct gtg tta agt ctt gaa cct gct ttt 942
   gct gaa cat tgg tca ggg gaa ttt gag aag tgg aaa gtg ggc ttt ttt 990
   cac cct ttg aaa aga gag gat gga ttc ttc acc aga act gac att 1035
   taaaaaaagt cagcgtggca cgttttagta tgtgtggcag atctaaagag acaatatttt 1095
   gatctcagga gtgtttattc ttgaaccatt ttcagaactc taagatttga gaaataataa 1155
   aatattgacc atccttcaaa gagaaaaaca cagggcgatc tttggcatag cctgtcattt 1215
   tgctcacatt tcacttctct ctctccaact tcagagcccc tgctgtggaa caggtgctgt 1275
   gctgggtggc aggggaggtc tctggctttt tttttttgat ctccgtctta acatctagcc 1335
   tactggagga agtgtattta atcatccact tatctgttaa caattatctc tgagggcccg 1395
   tcacattcag agaagattct aggttctcta caagtatcct ctcactgtgt acatactaaa 1455
   tcaacatcct gctggatttc ccccagacat ctcccttcat caccattgga gagtatcctc 1515
   taattgccag ccctattcac catactcatc tcatttgatc tggagttttc tgagagtgac 1575
   cgggggtggg atggacagga taatttagca agagtgtata agtaaaatct atataataaa 1635
   agttatctcc ctgtgccccc catgatctat tctttatgta gcagtctgaa tgagattttc 1695
   agaaacaaga accactttac cttagtctct tcttcttctt cttcttcttt tcttttcttt 1755
   ttttttagta ttatggggat ctgtttctgt tgcccagggt ggagtgcagt ggtatgatct 1815
   tggctcacag cagccttgaa ctcccgggct caagtggtcc tcctgcctct gcttccctag 1875
   tagctaggac tgcaggtttg tgccaccaca cctggctaat tgaaaaaaga aatttttttt 1935
   caatagagac agtgtcttgc tatgtcccca ggctggtctc aaactcctgg cctcaagtga 1995
   tcctcctgtc tcatcctccc aaagtgttgg aattacaggt gtgagctact atactcggcc 2055
   agtacccttc tcaaaacact tcagcacttc ccattgcact tgggttgaaa ttcccaccac 2115
   tcactggggc ccacaagact cttcaagact gaatccttgc tcaacattgt gacctgcccc 2175
   ctaccacctg cagcctcact tgctgtgctc cagccatgtg gatcttcctc ctgtctctaa 2235
   aactgcctca ggtcatttgc acctgctgtt cttcccaaag gctgtgtgat ttccatcagt 2295
   cagtcttagc tcgtatacct ccttggagac acctcttctg accaaccagt ccaaagaatc 2355
   tcctcttatc atgtcactct gttttattta tttatttaga gatggagtct cgctctgtca 2415
   cccaggctgg agtgcagtgg cgcgatctct gctcactgca agctccacct cctgggttca 2475
   tgccgttctc ctgcctcagc ctcctgagta actgggacta tgggcaccca ccactacacc 2535
   cggctaattt tttgtatttt tagtggggat ggggtttcac tgtgttagcc aggatggtct 2595
   tgatctcctg accttgtgat ctgcctgcct ccacctccca aagtgtttta tttattttaa 2655
   aggcatgtat cactctctga aaattagctt ctttcttctt tttccttgtt atcatccatt 2715
   tccccgaacc agaatagaag ttcctgaggc cagaacttct gtctctctgc ccctcactat 2775
   gtgtctctgg cacatacccc agtgcctgcc tgctctaaag taaaatctta gtaaatatta 2835
   ctgttgacta aataaatgaa taaatccctt ttaatgcccc tttggaagtt gccaagtaaa 2895
   gaataggatc cctttttaag attacacttt tggctattga tctgtgtgtc tggaacaaga 2955
   tacagtttga agatactacc atgggacatg acatcagttg agctgattaa ggttttagta 3015
   ataagaatcc aggatgtgtc cgggtgcggt gctcacgcct gtaatcctag cattttggga 3075
   gaccgaggcg ggcagatcac gaggtcagca gtttgagacc agcctgacca acatggtgaa 3135
   accccgtctc tactaaaaaa tacagaaatt agccgggtgt ggtggtgtcc acctgtagtc 3195
   ctagctactc aggaggctgg ggcaggagaa tttcttgaac ccgggaggcg gaggttgcag 3255
   tgagccgaga tcacaccagt gcactccagc ctgggcaaca gagcaagacc cagtctcagg 3315
   aaaaaaaaaa aaaa 3329
<210> 6
   <211> 2276
   <212> DNA
   <213> Homo sapiens
<220>
   <221> CDS
   <222> (103).. (486)
<400> 6
   ctgaactggg agtcaggtgg ttgacttgtg cctggctgca gtagcagcgg catctccctt 60
   gcacagttct cctcctcggc ctgcccaaga gtccaccagg cc atg gac gca gtg 114
   Met Asp Ala Val
   1
   gct gtg tat cat ggc aaa atc agc agg gaa acc ggc gag aag ctc ctg 162
   Ala Val Tyr His Gly Lys Ile Ser Arg Glu Thr Gly Glu Lys Leu Leu
   5 10 15 20
   ctt gcc act ggg ctg gat ggc agc tat ttg ctg agg gac agc gag agc 210
   Leu Ala Thr Gly Leu Asp Gly Ser Tyr Leu Leu Arg Asp Ser Glu Ser
   25 30 35
   gtg cca ggc gtg tac tgc cta tgt gtg ctg tat cac ggt tac att tat 258
   Val Pro Gly Val Tyr Cys Leu Cys Val Leu Tyr His Gly Tyr Ile Tyr
   40 45 50
   aca tac cga gtg tcc cag aca gaa aca ggt tct tgg agt gct gag aca 306
   Thr Tyr Arg Val Ser Gln Thr Glu Thr Gly Ser Trp Ser Ala Glu Thr
   55 60 65
   gca cct ggg gta cat aaa aga tat ttc cgg aaa ata aaa aat ctc att 354
   Ala Pro Gly Val His Lys Arg Tyr Phe Arg Lys Ile Lys Asn Leu Ile
   70 75 80
   tca gca ttt cag aag cca gat caa ggc att gta ata cct ctg cag tat 402
   Ser Ala Phe Gln Lys Pro Asp Gln Gly Ile Val Ile Pro Leu Gln Tyr
   85 90 95 100
   cca gtt gag aag aag tcc tca gct aga agt aca caa ggt act aca ggg 450
   Pro Val Glu Lys Lys Ser Ser Ala Arg Ser Thr Gln Gly Thr Thr Gly
   105 110 115
   ata aga gaa gat cct gat gtc tgc ctg aaa gcc cca tgaagaaaaa 496
   Ile Arg Glu Asp Pro Asp Val Cys Leu Lys Ala Pro
   120 125
   taaaacacct tgtactttat tttctataat ttaaatatat gctaagtctt atatattgta 556
   gataatacag ttcggtgagc tacaaatgca tttctaaagc cattgtagtc ctgtaatgga 616
   agcatctagc atgtcgtcaa agctgaaatg gacttttgta catagtgagg agctttgaaa 676
   cgaggattgg gaaaagtaat tccgtaggtt attttcagtt attatattta caaatgggaa 736
   acaaaaggat aatgaatact ttataaagga ttaatgtcaa ttcttgccaa atataaataa 796
   aaataatcct cagtttttgt gaaaagctcc atttttagtg aaatattatt ttatagctac 856
   taattttaaa atgtcttgct tgattgtatg gtgggaagtt ggctggtgtc ccttgtcttt 916
   gccaagttct ccactagcta tggtgtcata ggctcttttg ggatttttga agctgtatac 976
   tgtgtgctaa aacaagcact aaacaaagag tgaaggattt atgtttaatt ctgaaagcaa 1036
   ccttcttgcc tagtgttctg atattggaca gtaaaatcca cagaccaacc tggagttgaa 1096
   aatcttataa tttaaaatat gctctaaaca tgtttatcgt atttgatgct acaggatttg 1156
   aaattgtatt acaaatccaa tgaaatgagt ttttcttttc atttacctct gccccagttg 1216
   tttctactac atggaagacc tcattttgaa gggaaatttc agcagctgca gctcatgagt 1276
   aactgatttg taacaagcct ccttttaaag taaccctaca aaaccactgg aaagtttatg 1336
   gttgtattat tttttaaaaa aattccaagt gattgaaact tacacgagat acagaatttt 1396
   atgcggcatt ttcttctcac atttatattt ttgtgatttt gtgattgatt atatgtcact 1456
   ttgctacagg gctcacagaa ttcattcact caacaaacat aatagggcgc tgagggcata 1516
   gaagtaaaaa cacctggtcc ctgctctcag ttcactgtct tgttggacga gaaaacaata 1576
   acgataaaag acagtgaaag aaaataacga taaaagacag tgaaagaaaa taacaataaa 1636
   agacaaggaa aaaataacaa tgaaagttga taagtacatg ataagcgagg ttccccgtgt 1696
   gtaggtagat ctggtcttta gaggcagata gataggtcag tgcaaatact ctggtccatg 1756
   ggccatatga aaaggctaag cttcactgta aaataataac tgggaattct gggttgtgta 1816
   tgggtgttgg tgaacttggt tttaattagt gaactgctga gagacagagc tattctccat 1876
   gtactggcaa gacctgattt ctgagcattt aatatggatg ccgtgggagt acaaaagtgg 1936
   agtgtggcct gagtaatgca ttatgggtgg tttaccattt cttgaggtaa aagcatcaca 1996
   tgaacttgta aaggaattta aaaatcctac tttcataata agttgcatag gtttaataat 2056
   ttttaattat atggcttgag tttaaattgt aataggcgta actaatttta actctataat 2116
   gtgttcattc tggaataatc ctaaacatat gaattatgtt tgcatgttca cttccaagag 2176
   cctttttttg aaaaaaagct ttttttgaat catcaagtct ttcacattta aataaagtgt 2236
   ttgaaagctt tatttaaaaa aaaaaaaaaa aaaaaaaaaa 2276
<210> 7
   <211> 4343
   <212> DNA
   <213> Homo sapiens
<220>
   <221> CDS
   <222> (1)..(1029)
<400> 7
   atg gac gcc cca aaa gca gga tac gcc ttt gag tac ctt att gaa aca    48
   tta aat gac agt tca cat aag aag ttc ttc gat gta tct aaa ctt ggc    96
   acc aag tat gat gtt ctg cct tac tca ata cgg gtc ttg ttg gaa gct   144
   gct gta cga aat tgt gat ggc ttt tta atg aag aag gaa gat gtt atg   192
   aac att tta gac tgg aaa acc aaa caa agc aat gtt gaa gtg ccc ttt   240
   ttc cct gcc cgt gtt ctt ctt caa gat ttt act gga ata cca gca atg   288
   gtg gat ttt gct gct atg agg gag gca gtg aaa act ctt gga ggt gat   336
   cct gag aaa gtc cat cct gct tgt ccg aca gat ctt aca gtt gac cat 384
   tct tta caa att gac ttc agt aaa tgt gca ata cag aat gca cca aat 432
   cct gga ggt ggt gac ctg cag aaa gca gga aag ctc tct cca ctt aaa 480
   gtg cag cct aag aag ctt ccc tgc aga ggc cag act acc tgc cga gga 528
   tct tgt gat tct gga gaa cta ggc cga aac tca gga aca ttt tct tcg 576
   cag att gag aat aca ccc atc ctg tgt cct ttt cat ttg caa cca gtg 624
   cct gaa cct gaa aca gtg tta aaa aat caa gaa gta gaa ttc ggc aga 672
   aat cga gag agg ctt cag ttt ttt aag tgg agt tca aga gtt tta aag 720
   aat gtg gca gtg atc cct cct gga act gga atg gct cat caa ata aac 768
   tta gaa tat ttg tca aga gtg gtt ttt gaa gaa aaa gac ctc ctc ttc 816
   cca gac agt gta gtc ggc aca gat tca cac ata acg atg gtg aat ggt 864
   tta ggg att ctg ggg tgg ggg gtt gga ggc att gaa aca gaa gca gtt 912
   atg ctt ggt ctg cca gtt tct ctt act tta cca gag gtg gtt gga tgt 960
   gag tta act ggg tca tca aac cct ttt gtt aca tcc ata gat gtt gtt 1008
   ctt ggt att aca aag gta agt taaagttgtg gtagctctat gacttactga 1059
   acattatttt tataaaaatt gaagagctct atgagagcag ggatttgggt tcattactgc 1119
   atcctcaggt ctcttgacgt tagccacatc atcatagtta tcatagtaat aacaacaaac 1179
   agagcattta gtttgtacta ataaatacaa agaaatttgt tgtgttcact tatgttagct 1239
   catttagtcc ttataacaag cctgtgagat ggatactatt actattctca ttgtaactct 1299
   gagaaaacta aggtacagta gggtttagtg acttaccaaa gggtcgaagg cctgagtata 1359
   aggggtagag caaagattcc aggcagtcag attcttgagt catgtctaac cattatgcct 1419
   tattagtgcc ttgttgcctt aataaacact tgctggacta catatttttt ttctcttttt 1479
   taacttgaat taaaaaaaaa tgtttagcaa aagttgawtg tgtcgtcttt aattaaatta 1539
   tttgcccgtt agaaactgtt gctctactaa gtaatgcttt caaaaacatg gactgtagaa 1599
   atgtgatata tcatttttct gttgccgttt taacatttct ctggattatt atgtaaaaat 1659
   cttctctctg aatttttaaa atactggctt cagaacttca atacatacac tgagcttgtt 1719
   aagcatatta atacacaggc tcacggattt cctagtgaac aataatttgt aactcttctt 1779
   cctaaatgtc tggcctttgc taactttatt ttaatgatta aatcctattt tgttaaatga 1839
   atgtacctgg aaaatgttcc acatataatt ccaatttgag tcccaatctc agcatttttg 1899
   gttagattat tggtacgaag gctttctgga tactccagtg taaggaaatg ataatgcctc 1959
   cctctcagca tttggtattg atccttcttc cctaattaga aaagaatttg gcatcttaga 2019
   gaaattattg attcaacgta tgataccaaa agatcaagta gtaaattggg aattgcagga 2079
   ttattcctag aggaaaagga gtatcccatt atgtttttac agaaatcaat tctttacttt 2139
   agacatcctg aaaactaacg ctgcttttta gccttctcta gctgtttttt cctgacaata 2199
   ttactgtgtg ttttttgaca ttttagttta atgttaaaaa attaatctat tatatatgtt 2259
   tacatttatt gaatatattg attacttctt ttttgagatc ctgttccatt tgtgatcctt 2319
   ataggaataa tcctgtattg tttttttgat gagagcagca tttggtttgt aatatctaat 2379
   ctgtgtttct ttcatcctaa aaaataaaac cataggccgg gcgcggtggc tcacgcctgt 2439
   aatcccagca ctttgggagg ccgaggcggg tggatcatga ggtcaggaga tcgagaccat 2499
   cctggctaac aaggtgaaac cccgtctcta ctaaaaatac aaaaaattag ccgggcgcgg 2559
   tggcgggcgc ctgtagtccc agcttctcgg gaggctgagg caggagaatg gcgtgaaccc 2619
   gggaggcgga gcttgcagtg agccaagatt gcgccactgc agtccgcagt ccggcctggg 2679
   cgacagagcg agactctgty tcaaaaaaaa aaaaaaaaaa aaaaaaacca taaatgagga 2739
   aacgcatctt tacacttagg gtttgagttt ctgtatctat aaaaaagggt ttggattaag 2799
   tgatccctgg cacttataaa atgttagggc ttaatattat tcatagatcg aggatagttt 2859
   cattcttagt cgcctcctta gtcactcttc ctataccaat ctgagaccat tttacaattt 2919
   agaaaagaca aataactggt tgggttactt gatagtataa taaccaagaa aaataatttt 2979
   agaaggaatt aagtttgaaa ccacatgtta acaaattcta ccaaagtggg atttgcctgt 3039
   gattaaagat gctgtaaaca tttgggccag tagttataat ttgaaaaatg tttatagcca 3099
   atatataatt ttttatttaa atatacagtt tcatcagtct attagtattt cattaagtct 3159
   aagatgccat cagtggttag caaacaccac tgttttatgc actgctaaga aagaataaag 3219
   ggctgtgtgc agtggctcac acctgtggga cgccaaggca ggagcatcac ttgaggccag 3279
   aagttcaaga ccaacctggt caacattgta agaccctgtc tctacaaaaa aaaaaaagtt 3339
   aaaaattagc tgggtgcggt ggcacatgcc tgtagttcca gctactctgg aggctaaggt 3399
   gggaggattg ctagagccac ggtgttggaa gctgcaatga gctgtgacca caccactgcg 3459
   ctccagcgtg ggcaacagag tgagaccctg tttctaaaag aaagaaagaa aaaagggctg 3519
   ccacctaaac agacacacta ttgagttgag gtaccctgat ttcaaagaca tgaaaatgtt 3579
   aattatagcc accttgaagc tttcaggccc ctttctaccc tgaattaaca gtgacattgg 3639
   accagtcttc tctttacttc ttatcttaaa atacccccaa aaccagaatg agttgattca 3699
   taaggacaat gaaggatctc attccttcac catcactagt attggttaaa aattttattt 3759
   tatagttttc agacaatcgt tgctaatctt atctttgcaa ttttgtatgt gtttctgtgt 3819
   attccttata tagcacctca ggcaagtagg agtggctgga aagtttgttg agttttttgg 3879
   aagtggagtt tcacaattat ctatagttga tcgaactaca atagcaaaca tgtgtccgga 3939
   atatggtgct atcctcagct ttttccctgt tgacaatgtg acattaaaac atttagaaca 3999
   tacaggtaag aagataaaag atcactagaa taaacatgtt acatttccaa tgtgtttgat 4059
   aatattttat aaattactac cttatccatg ttatttacta ctcacaaaat tacattatgt 4119
   tgaaacaaca actttcaagc aaacatcaga tgtctttaaa gagtgttgtg tcctcaaacc 4179
   ctagttccct gtgacacatt gaaagcaatt taaaggaatt attcaaacca ttgatcctga 4239
   cttgactgtt tcccataatg atggatacct ccccctctac ttaggggtca taggtgcaat 4299
   ttaatggagt cagcccttaa acatattcac agcagtcccc ttct 4343
<210> 8
   <211> 155
   <212) DNA
   <213> Homo sapiens
<400> 8
   cacttataaa atgttagggc ttaatattat tcatagatcg aggatagttt cattcttagt 60
   cgcctcctta gtcactcttc ctataccaat ctgagaccat tttacaattt agaaaagaca 120
   aataactggt tgggttactt gatagtataa taacc 155
<210> 9
   <211> 278
   <212> DNA
   <213> Homo sapiens
<400> 9
   gaaggagaat atgaagaggt tagaaaagnt cnggnttctg ttggtgaaat gaaggatgaa 60
   ggggaagaga cattaaatta tcctgatact accattgact tgtctcacct tcaaccccaa 120
   aggtccatcc agaaattggc ttcaaaagag gaatcttcta attctagtga cagtaaatca 180
   cagagccgga gacatttgtc agccaaggaa agaagggaaa tgaaaaagaa aaaacttcca 240
   agtgactcag gagatttaga agcgttagag ggaaagga 278
<210> 10
   <211> 135
   <212> DNA
   <213> Homo sapiens
<400> 10
   ttctgacaat gagtaagaag aaagagggtc ttgccctttg gttattaaga tttatcatag 60
   agcaataata agtaaatcgg tgttatacca gcacagagat tagacaaata aaccaaggga 120
   ctggactaaa taagc 135
<210> 11
   <211> 197
   <212> DNA
   <213> Homo sapiens
<400> 11
   atggtaccca gtttcaaatt aacatggtta ttttacttgt gttcccaaat ttaacattag 60
   ggaatttttg gttgtgggtc tgttatcact agaaaaatat atatattggt gctgaagata 120
   attttgagat aattagacaa gacagtttag catttacaag aacaagtttg gcagttgaag 180
   aatctattta tatgact 197
<210> 12
   <211> 137
   <212> DNA
   <213> Homo sapiens
<400> 12
   ccaccgcacc tggctgatgc ttttctatct gacttctttc agaggaccct gaaagacact 60
   aagtggaatc tttccttgaa gtcttccaag ctaaaacaat tctctggaaa gatcacctct 120
   gttcagtcct ggtctct 137
<210> 13
   <211> 274
   <212> DNA
   <213> Homo sapiens
<400> 13
   cgtttacaga ttctcttgcg gctggcggtg gaactacaaa gggatcggtg cctatatcac 60
   aataccaaac ttgataataa tctagattct gtgtytctgc ttatagacca tgtttgtagt 120
   aggtaagagg aaaacttcct atattctgaa acagcctaac attttacaaa attttagttt 180
   tcttttttag agtcttatcc tgtagctata taacagttca tgtctgattt agcatttgtt 240
   cacgagtaaa gctggaacta tgaaaattga aaat 274
<210> 14
   <211> 171
   <212> DNA
   <213> Homo sapiens
<400> 14
   gattaggtga ccttccttga aragccacgg gtttcccata tagaaatgct attcattacc 60
   cgagtcacct angttcttac aaaggaagcg agaaaattgc ttttgttggg ccatgcccct 120
   tttgcanagg ttcctaagta tagtcgccan aattttttta atggcctaaa g 171
<210> 15
   <211> 161
   <212> DNA
   <213> Homo sapiens
<400> 15
   aggggcgctt gttctgctct cagcagattg gttacacgcg tcaggtggtg gcgatgactt 60
   aattcctagc ccaagaagaa tataatgtta aaactggtta tgtaattttt gtgcctctcc 120
   tttttaatgc agtatttagt tcagatgttg gcgatttttc a 161
<210> 16
   <211> 323
   <212> DNA
   <213> Homo sapiens
<400> 16
   tataaggwgg gaaccttact atctctaatg accttactga tgctgacttt aatactctgt 60
   gaaggttaga gttcagtgaa tgttacctag aaacagcccc ggctgtggaa tactttattc 120
   ttagccctat atttggggtt tggatgtcca ctgtgctggt tcccagagat agtaagggga 180
   tgagagtatt ggttacatct cctgacccac atacttaaga tccagatgaa caagacagtt 240
   ttcactcctg cttggtagaa cctatttgyk shaggaaaca gytcctaaag aatggttcta 300
   gccagaccct gtcgytacca gaa 323
<210> 17
   <211> 138
   <212> DNA
   <213> Homo sapiens
<400> 17
   agtatgacaa atagtttctg cctgattggt gagatttggg atgggccccc actttgtttc 60
   tctttctgca taaaaatttc aacattttta caaaattttc aaaaacttct cctcagtctg 120
   tacatctttg ttaatcag 138
<210> 18
   <211> 135
   <212> DNA
   <213> Homo sapiens
<400> 18
   tgatccccac aatttcttgt gattggtgag gaactataaa tgactcccat ccaagcttat 60
   accagaaaaa aggagcacat tttctacaaa ttatatcatt tttaatccat taccacatta 120
   ttttagggga actac 135
<210> 19
   <211> 219
   <212> DNA
   <213> Homo sapiens
<400> 19
   ctgagaggag ccatgtatac aaaccacttt ttctaacatg gtctttatta aactttgaat 60
   ataagtacac ctgctcgaag tgttcatcta tattatttaa gaacaagcaa ctgtaaaaca 120
   gtaaaatcac aaaaggtaag ttgttggaag acaacaaaaa agaattacta tatctgatcc 180
   tgcgtgttta ttttagaatc tgttaatagg cctacagct 219
<210> 20
   <211> 191
   <212> DNA
   <213> Homo sapiens
<400> 20
   acagtgagtg tggctgaaac ctaagctgaa ggaagggagg agcaggcact gccatgaggg 60
   gtccctggac agaaactctt cagcaggcct tgaagtttag ttcaggggct acatggaata 120
   ccactattta gcacacaggt gtgatctgag gtgagggact accttttcga tcttggtttt 180
   ctcatttatt t 191
<210> 21
   <211> 148
   <212> DNA
   <213> Homo sapiens
<400> 21
   ctggaggtga agggaaggaa agaaaggaaa aactatctac ctggcaggaa aagagataag 60
   ctcccaagaa caccaaagca gatgatgagt ctagctctac ccagccttcc tccccacgaa 120
   tccagatcat agtaagaaac tctgggct 148
<210> 22
   <211> 306
   <212> DNA
   <213> Homo sapiens
<400> 22
   ccaccaccag aaatgaacaa aaagcatttt acctaaaaat acaccagcaa aatgtactca 60
   gcttcaatca caaatacgac tgcttaaaac cgcagaaatt tcctcaacac tcagccttta 120
   tcactcagct ggattttttc cttcaacaat cactactcca agcattgggg aacacaactt 180
   ttaatcatac tccagtcgtt tcacaatgca ttctaatagc agcgggatca gaacagtact 240
   gcatttactt gccaacagaa cagacagacc tgaagtcaag acaactgcat tctctgtgaa 300
   gtctgt 306
<210> 23
   <211> 357
<212> DNA
   <213> Homo sapiens
<400> 23
   gtagcatttt ggcagaacca ttgttaatta aagggactty tggaccgcaa cyttaatgta 60
   ccagattatt gagcrgccca atgaatgctt cattctcatt gtttaaggtg ctgctttgat 120
   ttttttttca attctttgta ctatttttta ttttttggag aggcacatcc ccaaatttgg 180
   atgaggtatt tgttgataaa taattcatca atttccacaa tgcagacaaa aatgtctgcc 240
   cagagtggaa aaataaaaca agggggagaa gagtttgagt aacggagaag ttctgtggaa 300
   tcctagtgac aaaagttgag aaactacctt taaataagac agtgaggtaa caaatgt 357
<210> 24
   <211> 219
   <212> DNA
   <213> Homo sapiens
<100> 24
<210> 25
   <211> 251
   <212> DNA
   <213> Homo sapiens
<400> 25
   tgaaagggga atagaagcac aagagtcagt aatcaataac aaacaactca aggtgctcct    60
   tccttacact ggtgttcccc aaagtgaggt gaattgccag ccactgggag tcagggccag   120
   ttacataaga cattctcggt aagccccctt tgggtatccc aaataaggac tggggtgggt   180
   ttatgtgtag tccattatta acaactaaac gaacaaacct agtgaattgc aataaattca   240
   caccaacaga a                                                        251
<210> 26
   <211> 233
   <212> DNA
   <213> Homo sapiens
<400> 26
   gttgaaagag tccttggaag gcttttagac caaacccctc tgcatgctca arccttgggt    60
   acaggatttc taagaagtgg aacagtctcc aggggtgtgg arctcatcgc tcaaggcagg   120
   ttatcttatc tgaataattt tgtctgttga ctattgggat agttctcctt cagatgagct   180
   gaaatttcct ccatagcttc ctctattaaa cccaattcca cttctcaggg tca          233
<210> 27
   <211> 176
   <212> DNA
   <213> Homo sapiens
<400> 27
   caaaagcgct gaagttaagc attaatacgc cagattcatg atttatgatc agtatccaaa 60
   actccaacta caaacaatgc aaagtagtgc tcctcagtat tatttttgca attgttagta 120
   atgttaagca tcaaggaaaa taaaacacat cattgcacat tacagccgca aaaaac 176
<210> 28
   <211> 241
   <212> DNA
   <213> Homo sapiens
<400> 28
   agagagtaaa gcaagctatt ttgacagcaa cctaataaca gctgtcttct tccacttctt 60
   ggctaactca tcccccagat agccttcttt tctcttatca attccctgtt gcaacaataa 120
   taaatgccac acctgatgga gtcattaggc actttcctag tgacaagtgc ctaggacaga 180
   ggagaaaaca aagaaacact gacaaccact gaaaactgac atatcaggcc aggcatgtca 240
   c 241
<210> 29
   <211> 217
   <212> DNA
   <213> Homo sapiens
<400> 29
   gctggagagg tggtgatgtt gctgaataat tgctttttaa agctggaggg gacttccaag 60
   agtctctcat ttaagaaraa aaattaaaga cataattggt aacggttttg actgctgcag 120
   aggcaacact ttgctcacaa tcctacagat ctacttcacc tgtaactaca attttcctga 180
   agacatagaa gaaaaatcaa ttgttctaat ccatatg 217
<210> 30
   <211> 233
   <212> DNA
   <213> Homo sapiens
<400> 30
   aatcttagca taatgcttcc tgggaaattc tgaaattgat tccatttctg ccgttacaaa 60
   cacacacgaa gttcctagtt cactgggact tcctgatttg ttcttttagc ttgctccttc 120
   tcacctagaa gctctgttta tttctgagca accctggggc ttgtctcata ggacaggatt 180
   tatttatctc atcaaggctg agtgtgcctt aggaagtcat aaacataaaa aga 233
<210> 31
   <211> 228
   <212> DNA
   <213> Homo sapiens
<400> 31
   tatagacagg gtagggacga ttagcccctc gacaactttt cacaaatata cacacgttta 60
   actacctctc aggtcatgat aaagaccggc cgggcagaaa cactgtaatc ccagctactc 120
   gggagcctga ggcatgagaa tcacttgaac ctgggaggtg gaggttgcca tgagccgaga 180
   tcacgccatt gcactacagc cttggcgaca agagtgaaac tccatctg 228
<210> 32
   <211> 298
   <212> DNA
   <213> Homo sapiens
<400> 32
   gcttatgatt acaaacatcc ctcatatgaa aatctcagca tttnctggct gctgccttca 60
   atcgcttttt ctgaaatagg tatcccttga tgtcgactat ttgatttcag ccagtcgttt 120
   ctctctggca gtgctccctg caaatgtgtc ctttcaagaa aacaaaacct gcaagtggct 180
   tgtaatgtac catgacctta tcatgtgaag gacaaatggc tcttgtgctt attagatagc 240
   agatgaactg atgaactgaa ttcttggtct gaagctttga taaggtcaga tgtctttg 298
<210> 33
   <211> 291
   <212> DNA
   <213> Homo sapiens
<400> 33
   acttcgaagg gaaaaagagg aaggaaaagg actgttaata aaataacaaa ggcagcaatc 60
   agaatgaacc agagccagga cagcgtaaag gctaggttca cagtgagatg aaagaacctg 120
   aaaacaagtt taaaactcaa aagaggatta ttctcaagtt atactacagt gaaaaaacat 180
   ggaaaaacac aaaaaggaca ggcaataagg cacaggcata catacaaggc aaattgtaac 240
   acaatattta cttgcaaaag agcccacaga gacatgtcaa tgaagtcata g 291
<210> 34
   <211> 230
   <212> PRT
   <213> Homo sapiens
<400> 34
   Met Glu Asp Gly Phe Leu Asp Asp Gly Arg Gly Asp Gln Pro Leu His
   1 5 10 15
   Ser Gly Leu Gly Ser Pro His Cys Phe Ser His Gln Asn Gly Glu Arg
   20 25 30
   Val Glu Arg Tyr Ser Arg Lys Val Phe Val Gly Gly Leu Pro Pro Asp
           35                  40                  45
   Ile Asp Glu Asp Glu Ile Thr Ala Ser Phe Arg Arg Phe Gly Pro Leu
       50                  55                  60
   Ile Val Asp Trp Pro His Lys Ala Glu Ser Lys Ser Tyr Phe Pro Pro
   65                  70                  75                  80
   Lys Gly Tyr Ala Phe Leu Leu Phe Gln Asp Glu Ser Ser Val Gln Ala
                   85                  90                  95
   Leu Ile Asp Ala Cys Ile Glu Glu Asp Gly Lys Leu Tyr Leu Cys Val
               100                 105                 110
   Ser Ser Pro Thr Ile Lys Asp Lys Pro Val Gln Ile Arg Pro Trp Asn
           115                 120                 125
   Leu Ser Asp Ser Asp Phe Val Met Asp Gly Ser Gln Pro Leu Asp Pro
       130                 135                 140
   Arg Lys Thr Ile Phe Val Gly Gly Val Pro Arg Pro Leu Arg Ala Val
   145                 150                 155                 160
   Glu Leu Ala Met Val Met Asp Arg Leu Tyr Gly Gly Val Cys Tyr Ala
                   165                 170                 175
   Gly Ile Asn Thr Asp Pro Glu Leu Lys Tyr Pro Lys Gly Ala Gly Arg
                   180                 185                 190
   Val Ala Phe Ser Asn Gln Gln Ser Tyr Ile Ala Ala Ile Ser Ala Arg
               195                 200                 205
   Phe Val Gln Leu Gln His Gly Glu Ile Asp Lys Arg Val Ser Leu Ile
       210                 215                 220
   Leu His Phe Gly Lys Phe
   225                 230
<210> 35
   <211> 143
   <212> PRT
   <213> Homo sapiens
<400> 35 -
<210> 36
   <211> 104
   <212> PRT
   <213> Homo sapiens
<400> 36
   Met Pro His Met Leu Ser Gln Leu Ile Ala Gly Gly Val Ser Thr Ser
   1 5 10 15
   Cys Val Thr Ala Leu Gly Glu Glu Thr Gly Ala Trp Phe Pro Val Tyr
   20 25 30
   Leu Ser His Ala Ser Ser Pro Phe Ala Asp Leu Val Phe Cys Pro Phe
   35 40 45
   Ala Glu Ile Asn His Ser Gln Glu Tyr Asp Asn Met Arg Gly Pro Val
   50 55 60
   Ser Pro Pro Asn Lys Gln Phe Asn Leu Gly Val Ile Phe Gly Ile Pro
   65 70 75 80
   Asn Asn Cys Arg Phe Pro Thr Asp Asn Lys Ile Thr Glu Lys Gln Leu
   85 90 95
   Leu Gly Asn Val Leu Asn Tyr Pro
   100
<210> 37
   <211> 133
   <212> PRT
   <213> Homo sapiens
<400> 37
   Met Asn His Pro Trp His Val Cys Phe Leu Phe Lys Val Leu Arg Tyr
   1 5 10 15
   Tyr Pro Thr Ala Pro Ile Leu Lys Trp Thr His Thr Val Ser Cys Ser
   20 25 30
   Trp Cys Arg Ser Val Leu Arg Glu Val Val Gly Asn Val Ser Leu Ser
   35 40 45
   Glu Asn Phe Thr Ile Ser Ala Phe Cys Pro Glu Leu Thr Pro Phe Pro
   50 55 60
   Asp Gln Gly Thr Ser Thr Met Ile Ser Phe Leu Glu Lys Phe Asn Lys
   65 70 75 80
   Ser Lys Arg Glu Arg Leu Glu Leu Met Leu His Phe Tyr Ser Val Leu
   85 90 95
   Ser Leu Glu Pro Ala Val Ala Glu His Trp Ser Gly Glu Phe Glu Lys
   100 105 110
   Trp Lys Val Gly Phe Phe His Pro Leu Lys Arg Glu Asp Gly Phe Phe
   115 120 125
   Thr Arg Thr Asp Ile
   130
<210> 38
   <211> 133
   <212> PRT
   <213> Homo sapiens
<400> 38
<210> 39
   <211> 128
   <212> PRT
   <213> Homo sapiens
<400> 39
   Met Asp Ala Val Ala Val Tyr His Gly Lys Ile Ser Arg Glu Thr Gly
   1 5 10 15
   Glu Lys Leu Leu Leu Ala Thr Gly Leu Asp Gly Ser Tyr Leu Leu Arg
   20 25 30
   Asp Ser Glu Ser Val Pro Gly Val Tyr Cys Leu Cys Val Leu Tyr His
   35 40 45
   Gly Tyr Ile Tyr Thr Tyr Arg Val Ser Gln Thr Glu Thr Gly Ser Trp
   50 55 60
   Ser Ala Glu Thr Ala Pro Gly Val His Lys Arg Tyr Phe Arg Lys Ile
   65 70 75 80
   Lys Asn Leu Ile Ser Ala Phe Gln Lys Pro Asp Gln Gly Ile Val Ile
   85 90 95
   Pro Leu Gln Tyr Pro Val Glu Lys Lys Ser Ser Ala Arg Ser Thr Gln
   100 105 110
   Gly Thr Thr Gly Ile Arg Glu Asp Pro Asp Val Cys Leu Lys Ala Pro
   115 120 125
<210> 40
   <211> 343
   <212> PRT
   <213> Homo sapiens
<400> 40
<210> 41
   <211> 305.
   <212> DNA
   <213> Homo sapiens
<400> 41
   tcatgaagtg aagccaactg tttagactag aatgttatga gattaaaccc acnnnnnntt 60
   attcatagac ataaaccctc attttaatta gtggatctgg atttttgtca tatgtggaat 120
   cataatttaa acaaaatcaa ctaagatgat ccaagttcca cacaactgca cttcaatatt 180
   caagtcggtg tgaagatgcc tgactactgc gtcacaagat tctgagctgt cgtaaaaagc 240
   ctggctcgtg gtttctattt atagtgtaca catgttgggt tataatcaca aacctggaac 300
   tctgt 305
<210> 42
   <211> 256
   <212> DNA
   <213> Homo sapiens
<400> 42
   gaaaccacgg cttacaccta gagacagcat tcagatatag acgggatact tgtgttagtc 60
   agttccttta taacaggtga atctctctcc cactgcttca acactgcgtg acaaagccaa 120
   ttgggaagca gctttacaaa tgtgacttga cttggggatc ttcttgatac tttgccatgg 180
   caaggaacaa gccgcctgaa ctaaatgcca ctccatttga ttccacgctt aaagtaacca 240
   tgcaaccgac tatagt 256
<210> 43
   <211> 244
   <212> DNA
   <213> Homo sapiens
<400> 43
   tactcttcaa ccatgatttt tctctgatgg cctgtgtgaa cagattaatg gtgtccatct 60
   aattccttcc ccactggggg aaagcaaatc atcaggccca ttgcaaaaac tgctcttggt 120
   tgagcttcct gccttaaatc atacccacag tgaatggcgt ccctttatca ccgctaatga 180
   ctctgacatc tctctccact cacatgtgag cctcctcagc tctcgnnaaa caagtcngtc 240
   tcgg 244
<210> 44
   <211> 258
   <212> DNA
   <213> Homo sapiens
<400> 44
   tctcagaaaa ctccagatca aatgagatga gtatggtgnn nagggctggc aattagagga 60
   tactctccaa tggtgatgaa gggagatgtc tgggggaaat ccagcaggat gttgatttag 120
   tatgtacaca gtgagaggat acttgtagag aacctagaat cttctctgaa tgtgacgggc 180
   cctcagagat aattgttaac agataagtgg atgattaaat acacttcctc cagtaggcta 240
   gatgttaaga cggagatc 258
<210> 45
   <211> 26
   <212> DNA
   <213> Artificial Sequence
<220>
   <223> Description of Artificial Sequence:Synchetic DNA
<400> 45
   ggacttaata ttattcatag atcgag 26
<210> 45
   <211> 26
   <212> DNA
   <213> Artificial Sequence
<220>
   <223> Description of Artificial Sequence Synthetic DNA
<400> 46
   gttattatac tatcaagtaa cccaac 26
<210> 47
   <211> 25
   <212> DNA
   <213> Artificial Sequence
<220>
   <223> Description of Artificial Sequence:Synthetic DNA
<400> 47
   gtggatctgg atttttgtca tatgt 25
<210> 48
   <211> 25
   <212> DNA
   <213> Artificial Sequence
<220>
   <223> Description of Artificial Sequence:Synthetic DNA
<400> 48
   gtttgtgatt ataacccaac atgtg 25
<210> 49
   <211> 25
   <212> DNA
   <213> Artificial Sequence
<220>
   <223> Description of Artificial Sequence:Synthetic DNA
<400> 49
   gaaggggaag agacattaaa ttatc 25
<210> 50
   <211> 24
   <212> DNA
   <213> Artificial Sequence
<220>
   <223> Description of Artificial Sequence:Synthetic DNA
<400> 50
   gcttctaaat ctcctgagtc actt 24
<210> 51
   <211> 24
   <212> DNA
   <213> Artificial Sequence
<220>
   <223> Description of Artificial Sequence:Synthetic DNA
<400> 51
   gacaatgagt aagaagaaag aggg 24
<210> 52
   <211> 24
   <212> DNA
   <213> Artifice Sequence
<220>
   <223> Description of Artificial Sequence:Synthetic DNA
<400> 52
   gtccagtccc ttggtttatt tgtc 24
<210> 53
   <211> 25
   <212> DNA
   <213> Artificial Sequence
<220>
   <223> Description of Artificial Sequence:Synthetic DNA
<400> 53
   ggtacccagt ttcaaatcaa catgg 25
<210> 54
   <211> 25
   <212> DNA
   <213> Artificial Sequence
<220>
   <223> Description of Artificial Sequence:Synthetic DNA
<400> 54
   gattcttcaa ctgccaaact tgttc 25
<210> 55
   <211> 24
   <212> DNA
   <213> Artificial Sequence
<220>
   <223> Description of Artificial Sequence:Synthetic DNA
<400> 55
   gctgatgctt ttctatctga cttc 24
<210> 56
   <211> 22
   <212> DNA
   <213> Artificial Sequence
<220>
   <223> Descripton of Artificial Sequence:Synthetic DNA
<400> 56
   gaccaggact gaacagaggt ga 22
<210> 57
   <211> 25
   <212> DNA
   <213> Artificial Sequence
<220>
   <223> Description of Artificial Sequence:Synthetic DNA
<400> 57
   gcttatagac catgtttgta gtagg 25
<210> 58
   <211> 25
   <212> DNA
   <213> Artificial Sequence
<220>
   <223> Description of Artificial Sequence:Synthetic DNA
<400> 58
   gtgaacaaat gctaaatcag acatg 25
<210> 59
   <211> 22
   <212> DNA
   <213> Artificial Sequence
<220>
   <223> Description of Artificial Sequence:Synthetic DNA
<400> 59
   gccacgggtt tcccatatcg aa 22
<210> 60
   <211> 24
   <212> DNA
   <213> Artificial Sequence
<220>
   <223> Description of Artificial Sequence:Synthetic DNA
<400> 60
   gactatactt aggaacctct gcaa 24
<210> 61
   <211> 24
   <212> DNA
   <213> Artificial Sequence
<220>
   <223> Description of Artificial Sequence:Synthetic DNA
<400> 61
   gttctgctct cagcagattg gtta 24
<210> 62
   <211> 24
   <212> DNA
   <213> Artificial Sequence
<220>
   <223> Description of Artificial Sequence:Synthetic DNA
<400> 62
   gccaacatct gaactaaala ctgc 24
<210> 63
   <211> 25
   <212> DNA
   <213> Artificial Sequence
<220>
   <223> Description of Artificial Sequence:Synthetic DNA
<400> 63
   gttcagtgaa tgttacctag aaaca 25
<210> 64
   <211> 24
   <212> DNA
   <213> Artificial Sequence
<220>
   <223> Description of Artificial Sequence:Synthetic DNA
<400> 64
   ggagtgaaaa ctgtcttgtt catc 24
<210> 65
   <211> 25
   <212> DNA
   <213> Artificial Sequence
<220>
   <223> Description of Artificial Sequence:Synthetic DNA
<400> 65
   gtatgacaaa tagtttctgc ctgat 25
<210> 66
   <211> 25
   <212> DNA
   <213> Artificial Sequence
<220>
   <223> Description of Artificial Sequence:Synthetic DNA
<400> 66
   gattaacaaa gatgtacaga ctgag 25
<210> 67
   <211> 24
   <212> DNA
   <213> Artificial Sequence
<220>
   <223> Description of Artificial Sequence:Synthetic DNA
<400> 67
   gagacagcat tcagatatag acgg 24
<210> 68
   <211> 22
   <212> DNA
   <213> Artificial Sequence
<220>
   <223> Description of Artificial Sequence:Synthetic DNA
<400> 68
   gcgtggaatc aaatggagtg gc 22
<210> 69
   <211> 24
   <212> DNA
   <213> Artificial Sequence
<220>
   <223> Description of Artificial Sequence:Synthetic DNA
<400> 69
   gatggcctgt gtgaacagat taat 24
*<210>* 70
   <211> 24
   <212> DNA
   <213> Artificial Sequence
<220>
   <223> Description of Artificial Sequence:Synthetic DNA
<400> 70
   gagagagatg tcagagtcat tagc 24
<210> 71
   <211> 24
   <212> DNA
   <213> Artificial Sequence
<220>
   <223> Description of Artificial Sequence:Synthetic DNA
<400> 71
   gatccccca atttcttgtg attg 24
<210> 72
   <211> 25
   <212> DNA
   <213> Artificial Sequence
<220>
   <223> Description of Artificial Sequence:Synthetic DNA
<400> 72
   gttcccctaa aataatgtgg taatg 25
<210> 73
   <211> 23
   <212> DNA
   <213> Artificial Sequence
<220>
   <223> Description of Artificial Sequence:Synthetic DNA
<400> 73
   gaggatactc tccaatggtg atg 23
<210> 74
   <211> 24
   <212> DNA
   <213> Artificial Sequence
<220>
   <223> Description of Artificial Sequence.Synthetic DNA
<400> 74
   gtcttaacat ctagcctact ggag 24
<210> 75
   <211> 24
   <212> DNA
   <213> Artificial Sequence
<220>
   <223> Description of Artificial Sequence:Synthetic DNA
<400> 75
   gagaggagcc atgtatacaa acca 24
<210> 76
   <211> 26
   <212> DNA
   <213> Artificial Sequence
<220>
   <223> Description of Artificial Sequence:Synthetic DNA
<400> 76
   gcacgcagga tcagaatag taattc 26
<210> 77
   <211> 24
   <212> DNA
   <213> Artificial Sequence
<220>
   <223> Description of Artificial Sequence:Synthetic DNA
<400> 77
   gctgaaacct aagctgaagg aagg 24
<210> 78
   <211> 22
   <212> DNA
   <213> Artificial Sequence
<220>
   <223> Description of Artificial Sequence:Synthetic DNA
<400> 78
   gtcccctcacc tcagatcaca cc 22
<210> 79
   <211> 24
   <212> DNA
   <213> Artificial Sequence
<220>
   <223> Description of Artificial Sequence:Synthetic DNA
<400> 79
   gctatctacc tggcaggaaa agag 24
<210> 80
   <211> 25
   <212> DNA
   <213> Artificial Sequence
<220>
   <223> Descripticn of Artificial Sequence: Synthetic DNA
<400> 80
   gagtttctta ctatgatctg gattc 25
<210> 81
   <211> 25
   <212> DNA
   <213> Artificial Sequence
<220>
   <223> Description of Artificial Sequence:Synthetic DNA
<400> 81
   gcaaaatga ctcagcttca atcac 25
<210> 82
   <211> 24
   <212> DNA
   <213> Artificial Sequence
<220>
   <223> Description of Artificial Sequence:Synthetic DNA
<400> 82
   gtaaatgcag taccgttctg atcc 24
<210> 83
   <211> 25
   <212> DNA
   <213> Artificial Sequence
<220>
   <223> Description of Artificial Sequence:Synthetic DNA
<100> 83
   gaatgcttca ttctcattgt ttaagg 26
<210> 84
   <211> 24
   <212> DNA
   <213> Artificial Sequence
<220>
   <223> Description of Artificial Sequence:Synthetic DNA
<400> 84
   gtcactagga ttccacagaa cttc 24
<210> 85
   <211> 22
   <212> DNA
   <213> Artificial Sequence
<220>
   <223> Description of Artificial Sequence Synthetic DNA
<400> 85
   gaggtagggc ttcccttcgc ta 22
<210> 86
   <211> 25
   <212> DNA
   <213> Artificial Sequence
<220>
   <223> Description or Artificial Sequence:Synthetic DNA
<400> 86
   gcataacaag tgacagggtt agtta 25
<210> 87
   <211> 22
   <212> DNA
   <213> Artificial Sequence
<220>
   <223> Description of Artificial Sequence:Syntnetic DNA
<400> 87
   ggtgctcct ccttacactg gt 22
<210> 88
   <211> 23
   <212> DNA
   <213> Artificial Sequence
<220>
   <223> Description of Artificial Sequence:Synthetic DNA
<400> 88
   gactacacat aaacccaccc cag 23
<210> 89
   <211> 24
   <212> DNA
   <213> Artificial Sequence
<220>
   <223> Description of Artificial Sequence:Synthetic DNA
<400> 89
   gggtacagga tttctaagaa gtgg 24
<210> 90
   <211> 25
   <212> DNA
   <213> Artificial Sequence
<220>
   <223> Description of Artificial Sequence:Synthetic DNA
<400> 90
   ggagaaaatt tcagctcatc tgaag 25
<210> 91
   <211> 24
   <212> DNA
   <213> Artificial Sequence
<220>
   <223> Description of Artificial Sequence:Synthetic DNA
<400> 91
   gctgaagtta agcattaata cgcc 24
<210> 92
   <211> 23
   <212> DNA
   <213> Artificial Sequence
<220>
   <223> Description of Artificial Sequence:Synthetic DNA
<400> 92
   gcggctgtaa tgtgcaatga tgt 23
<210> 93
   <211> 24
   <212> DNA
   <213> Artificial Sequence
<220>
   <223> Description of Artificial Sequence:Synthetic DNA
<400> 93
   gacagcaacc taataacagc tgtc 24
<210> 94
   <211> 22
   <212> DNA
   <213> Artificial Sequence
<220>
   <223> Description of Artificial Sequence:Synthetic DNA
<400> 94
   gtcctaggca cttgtcacta gg 22
<210> 95
   <211> 22
   <212> DNA
   <213> Artificial Sequence
<220>
   <223> Description of Artificial Sequence:Synthetic DNA
<400> 95
   gaggggactt ccaagagtct ct 22
<210> 96
   <211>
   <212> DNA
   <213> Artificial Sequence
<220>
   <223> Description of Artificial Sequence:Synthetic DNA
<400> 96
   gtcttcagga aaattgtagt tacag 25
<210> 97
   <211> 24
   <212> DNA
   <213> Artificial Sequence
<220>
   <223> Description of Artificial Sequence:Synthetic DNA
<400> 97
   gttacaaaca cacacgaagt tcct 24
<210> 98
   <211> 22
   <212> DNA
   <213> Artificial Sequence
<220>
   <223> Description of Artificial Sequence:Synthetic DNA
<400> 98
   gacttcctaa ggcacactca gc 22
<210> 99
   <211> 24
   <212> DNA
   <213> Artificial Sequence
<220>
   <223> Description of Artificial Sequence:Synthetic DNA
<100> 99
   gtttaactac ctctcaggtc atga 24
<210> 100
   <211> 22
   <212> DNA
   <213> Artificial Sequence
<220>
   <223> Description of Artificial Sequence:Synthetic DNA
<400> 100
   gtcgccaagg ctgtagtgca at 22
<210> 101
   <211> 24
   <212> DNA
   <213> Artificial Sequence
<220>
   <223> Description of Artificial Sequence:Synthetic DNA
<400> 101
   gaaataggta tcccttgatg tcga 24
<210> 102
   <211> 24
   <212> DNA
   <213> Artificial Sequence
<220>
   <223> Description of Artificial Sequence:Synthetic DNA
<400> 102
   gaccaagaat tcagttcatc agtt 24
<210> 103
   <211> 22
   <212> DNA
   <213> Artificial Sequence
<220>
   <223> Description of Artificial Sequence:Synthetic DNA
<400> 103
   gaatgaacca gagccaggac ag 22
<210> 104
   <211> 22
   <212> DNA
   <213> Artificial Sequence
<220>
   <223> Description of Artificial Sequence:Synthetic DNA
<400> 104
   gccttgtatg tatgcctgtg cc 22
<210> 105
   <211> 21
   <212> DNA
   <213> Artificial Sequence
<220>
   <223> Description of Artificial Sequence:Synthetic DNA:
<400> 105
   aagagtccac caggccatgg a 21
<210> 106
   <211> 23
   <212> DNA
   <213> Artificial Sequence
<220>
   <223> Description of Artificial Sequence:Synthetic DNA
<400> 106
   taccttgtgt acttctagct gag 23
<210> 107
   <211> 17
   <212> DNA
   <213> Artificial Sequence
<220>
   <223> Description of Artificial Sequence:Synthetic DNA
<400> 107
   gtttttttt tttttta 17
<210> 108
   <211> 17
   <212> DNA
   <213> Artificial Sequence
<220>
   <223> Description of Artificial Sequence:Synthetic DNA
<400> 108
   gttttttttt ttttttg 17
<210> 109
   <211> 17
   <212> DNA
   <213> Artificial Sequence
<220>
   <223> Description of Artificial Sequence:Synthetic DNA
<400> 109
   gttttttttt ttttttc 17
<210> 110
   <211> 18
   <212> DNA
   <213> Artificial Sequence
<220>
   <223> Description of Artificial Scquence:Synthetic DNA
<400> 110
   cagagtgatg gatatcaa 18
<210> 111
   <211> 22
   <212> DNA
   <213> Artificial Sequence
<220>
   <223> Description of Artificial Sequence:Synthetic DNA
<400> 111
   atgaaagtgc cagtgtgcca tg 22
<210> 112
   <211> 22
   <212> DNA
   <213> Artificial Sequence
<220>
   <223> Description of Artificial Sequence:Synthetic DNA
<400> 112
   cccatcacca tcttccagga gc 22
<210> 113
   <211> 26
   <212> DNA
   <213> Artificial Sequence
<220>
   <223> Description of Artificial Sequence:Synthetic DNA
<400> 113
   ttcaccacct tcttgatgtc atcata 26
<210> 114
   <211> 15
   <212> PRT
   <213> Artificial Sequence
<220>
   <223> Description of Artificial Sequence:Synthetic Peptide
<400> 114
   Cys Pro Leu Lys Arg Glu Asp Gly Phe Phe Thr Arg Thr Asp Ile
   1 5 10 15
<210> 115
   <211> 16
   <212> PRT
   <213> Artificial Sequence
<220>
   <221> MOD_RES
   <222> (16)
   <223> AMIDATION, GluAmide
<400> 115
   Cys Ser Phe Leu Glu Lys Phe Asn Lys Ser Lys Arg Glu Arg Leu Xaa
   1 5 10 15
<210> 116
   <211> 15
   <212> PRT
   <213> Artificial Sequence
<220>
   <221> M0D_RES
   <222> (15)
   <223> AMIDATION. GlyAmide
<400> 116
   Cys Ala Glu His Trp Ser Gly Glu Phe Glu Lys Trp Lys Val Xaa
   1 5 10 15
<210> 117
   <211> 16
   <212> PRT
   <213> Artificial Sequence
<220>
   <223> Description of Artificial Sequence:Synthetic Peptide
<400> 117
<210> 118
   <211> 15
   <212> PRT
   <213> Artificial Sequence
<220>
   <223> Description of Artificial Sequence:Synthetic Peptide
<400> 118
   Cys Arg Leu Met Lys Arg Lys Thr Gly Glu Ser Leu Leu Ser Ser
   1 5 10 15
<210> 119
   <211> 14
   <212> PRT
   <213> Artificial Sequence
<220>
   <223> Description of Artificial Sequence:Synthetic Peptide
<400> 119
   Cys Thr Ser Ile Asp Val Val Leu Gly Ile Thr Lys Val Ser
   1 5 10
<210> 120
   <211> 16
   <212> PRT
   <213> Artificial Sequence
<220>
   <221> MOD_RES
   <222> (16)
   <223> AMIDATION LysAmide
<400> 120
   Cys Ser Ala Glu Thr Ala Pro Gly Val His Lys Arg Tyr Phe Arg Xaa
   1 5 10 15
<210> 121
   <211> 16
   <212> PRT
   <213> Artificial Sequence
<220>
   <223> Description of Artificial Sequence:Synthetic Peptide
<400> 121
   Cys Lys Ile Thr Glu Lys Gln Leu Leu Gly Asn Val Leu Asn Tyr Pro
   1 5 10 15

## Description

The present invention relates to a novel DNA whose expression level fluctuates in leukocytes of IgA nephropathy patients in comparison with leukocytes of healthy persons, a novel protein encoded by the DNA, an antibody recognizing the protein, a method for detecting the protein or the DNA, and methods of diagnosis of IgA nephropathy.

IgA nephropathy is a chronic glomerulonephritis which is characterized in that an IgA immune complex considered to be derived from blood deposits in glomerulus of the kidney. In Japan, the IgA nephropathy occupies 30% or more of primary renal diseases, having the highest frequency as a single renal disease, and 15 to 30% of the disease becomes renal failure due to poor prognosis. However, since the cause of the disease of IgA nephropathy is still unclear, a fundamental therapeutic method has not been found. Additionally, definite diagnosis of IgA nephropathy imposes heavy burden on patients, because the method is carried out by taking out a portion of the kidney by biopsy and recognizing deposition of the IgA immune complex in mesangium by means of immunological staining.

It has been reported that about 50% of the patients with IgA nephropathy have a high blood IgA level [Diseases of the Kidney, 5th edition (1993) , Nephron 29, 170 (1981)]. It is considered that B cells relate to the production of IgA in blood and T cells relate to the regulation of the production. Furthermore, it has been reported that the production of cytokine, such as interleukin 4, interleukin 5, interleukin 6 or TGF-β (transforming growth factor-β), is high in peripheral T cells of IgA nephropathy patients in comparison with healthy persons [Clinical & Experimental Immunology, 103, 125 (1996), Kidney International, 46, 862 (1994)] and that integrin, such as VLA (very late activation)-4 and VLA-5, are strongly activated in peripheral lymphocytes of IgA nephropathy patients [Nephrology, Dialysis, Transplantation, 10, 1342 (1995)]. On the basis of these facts, it is considered that, in IgA nephropathy, the production of. IgA becomes excess due to abnormality in the immune system, the resulting IgA immune complex in blood deposits on the glomerulus, and activation of the complement system caused thereby and the like exert influence upon disorders of the glomerulus, but the cause of IgA nephropathy has not been reported.

Elucidation of the cause of IgA nephropathy and its treatment or diagnosis which can reduce a burden on patients are expected.

A novel DNA related to IgA nephrophathy, a method for isolating the DNA, a novel protein related to IgA nephropathy, a method for producing the protein, an antibody recognizing the protein, and a therapeutic drug and a diagnostic drug using the above-described protein, DNA or antibody are desired.

The present invention is useful for at least some of these objections.

The present invention relates to the following (1) to (10):
(1) A DNA comprising a nucleotide sequence selected from the group consisting of:
   (a) a DNA having the nucleotide sequence of SEQ ID NO:1;
   (b) a DNA encoding a protein comprising the amino acid sequence of SEQ ID NO:34.
(2) A vector comprising the DNA of (1).
(3) A non-human transformant comprising the vector of (2).
(4) A method for producing a protein encoded by the DNA of (1) comprising: culturing the transformant of (3) in a medium to produce and accumulate said protein in the culture; and recovering said protein from the resulting culture.
(5) A protein encoded by the DNA of (1) or obtainable by the method of (4).
(6) An antibody which specifically recognizes a protein consisting of the amino acid sequence of SEQ ID NO:34.
(7) A method for detecting mRNA of an IgA nephropathy-related gene using the DNA of (1).
(8) A method for immunologically detecting the protein of (5) using the antibody of (6).
(9) A diagnostic composition comprising the DNA of (1), the vector of (2), the protein of (5) or the antibody of (6) and, optionally, a diagnostically acceptable carrier.
(10) Use of the DNA of (1), the vector of (2), the protein of (5) or the antibody of (6) for the preparation of a diagnostic composition for diagnosing IgA nephropathy.

The DNA of the present invention is a IgA nephropathy-related DNA. The DNA comprises a nucleotide sequence represented by SEQ ID NO:1. Further described is, a DNA which hybridizes with the DNA under stringent conditions.

The DNA which hybridizes under stringent conditions with a DNA comprising a nucleotide sequence represented by SEQ ID NO:1 means a DNA which is obtained by colony hybridization, plaque hybridization, Southern blot hybridization or the like using, as a probe, a DNA comprising a nucleotide sequence selected from the nucleotide sequence represented by SEQ ID NO:1. Examples include DNA which can be identified by carrying out hybridization at 65°C in the presence of 0.7-1.0 M NaCl using a filter on which a DNA prepared from colonies or plaques is immobilized, and then washing the filter with 0.1 x to 2 x SSC solution (the composition of 1 x SSC comprises 150 mM sodium chloride and 15 mM sodium citrate) at 65°C.

The hybridization can be carried out in accordance with known methods described in, for example, Molecular Cloning, A Laboratory Manual, Second Edition, Cold Spring Harbor Laboratory Press (1989) (referred to as "Molecular *Cloning,* 2nd ed." hereinafter), Current Protocols in Molecular Biology, John Wiley & Sons (1987-1997) (referred to as "Current *Protocols in Molecular Biology*" hereinafter), DNA Cloning 1: Core Techniques, A Practical Approach, Second Edition, Oxford University (1995) or the like. Specific examples of the DNA which can be hybridized include a DNA having a homology of 60% or more with a nucleotide sequence represented by SEQ ID NO:1, preferably a DNA having a homology of 80% or more, and more preferably a DNA having a homology of 95% or more.

Also, the DNA disclosed in the present invention includes an oligonucleotide and antisense oligonucleotide containing a partial sequence of the IgA nephropathy-related DNA.

Examples of the oligonucleotide include oligonucleotides comprising a sequence identical to a sequence of continuous 5 to 60 residues, preferably continuous .10 to 50 residues, in a nucleotide sequence represented by SEQ ID NO:1. Examples of the antisense oligonucleotide include antisense oligonucleotides of the oligonucleotides. Specific examples include oligonucleotides comprising a nucleotide sequence selected from the nucleotide sequences represented by SEQ ID NO: 47 and NO: 48.

The protein of the present invention has an activity related to IgA nephropathy. The protein comprises an amino acid sequence represented by SEQ ID NO:34. Further described is a protein comprising an amino acid sequence in which one or several amino acids are deleted, substituted or added in the amino acid sequence of said protein and having an activity related to IgA nephropathy.

The protein comprising an amino acid sequence in which one or several amino acids are deleted, substituted or added in the amino acid sequence of the protein that has an amino acid sequence represented by SEQ ID NO:34 and has an activity related to IgA nephropathy can be prepared in accordance with known methods described in, for example, Molecular Cloning, 2nd ed., Current Protocols in Molecular Biology, Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985) and the like. The number of the amino acids which are deleted, substituted or added is not particularly limited; however, they are preferably one to decades, more preferably one to several, amino acids. Also, in order that the polypeptide of the present invention has an activity relating to IgA nephropathy, it is preferred that they have a homology of at least 60% or more, generally 80% or more, and particularly 95% or more, with an amino acid sequence selected from the amino acid sequences represented by SEQ ID NO:34 to NO:40.

Examples of the antibody of the present invention include antibodies which recognize the above-described proteins.

The present invention is described in detail.

### 1. Preparation of IgA nephropathy-related DNA

Taking note of the difference in the expression quantity of mRNA in leukocytes between patients with IgA nephropathy and healthy persons, the IgA nephropathy-related DNA is isolated using the differential display method [FEBS Letters, 351, 231 (1994)]. That is, an amplified cDNA fragment of a novel gene (referred to as "IgA nephropathy-related gene" hereinafter) whose expression level increases or decreases significantly in leukocytes of a patient with IgA nephropathy as compared with leukocytes of a healthy person is obtained by subjecting total RNA or mRNA extracted from cells to the polymerase chain reaction (PCR) using various primers.

This method is described below.

Total RNA or mRNA is prepared from leukocytes of patients with IgA nephropathy and leukocytes of healthy persons.

Examples of the method for the preparation of total RNA include guanidine thiocyanate-cesium trifluoroacetate method [Methods in Enzymol., 154, 3 (1987)] and the like.

Examples of the method for preparing poly(A)⁺ RNA from total RNA include oligo(dT)-immobilized cellulose column method (*Molecular Cloning*, 2nd ed.) and the like.

The mRNA can also be prepared using a kit, such as Fast Track mRNA Isolation Kit (manufactured by Invitrogen), Quick Prep mRNA Purification Kit (manufactured by Pharmacia) or the like.

Using an anchor primer, cDNA is synthesized in the usual way from the RNA extracted by the above-described method from leukocytes of a patient with IgA nephropathy or leukocytes of a healthy person, and then the cDNA is amplified by subjecting it to PCR using an anchor primer having a 5'-end labeled with fluorescence and an arbitrary primer.

The anchor primer is a primer in which an oligonucleotide of adenine, guanine or cytosine, excluding thymidine, is added to the 3'-end of an oligo(dT) sequence which hybridizes with a 3'-end poly (A) sequence of mRNA, and the primer can be synthesized using DNA Synthesizer Model 392 (manufactured by Perkin-Elmer) or the like.

The arbitrary primer is an oligonucleotide which amplifies various cDNA sequences and can yield a large number of amplified DNA fragments by a single reaction. Examples include OPD-1 to 20, OPE-1 to 20, OPV-1 to 20 (manufactured by Operon Technologies), and the like. Preferably, the arbitrary primer may have a length of about 10 bases.

Each of the DNA amplified by PCR is subjected to polyacrylamide gel electrophoresis, and the amount of fluorescence of the resulting bands is measured using Fluoro Imager (manufactured by Molecular Dynamics).

By comparing intensities of fluorescence of respective bands, a portion of the gel, which corresponds to the position of band where the intensities of fluorescence are fluctuated between the IgA nephropathy patient and healthy person, is cut off and the DNA fragment contained in the gel is amplified by PCR.

The nucleotide sequence of the DNA is determined by inserting the amplified DNA fragment into a vector, directly or after blunt-ending its termini using a DNA polymerase, in the usual way and then analyzing it by a usually used nucleotide sequence analyzing method such as the dideoxy method of Sanger et al. [Proc. Natl. Acad. Sci. USA, 74, 5463 (1977)] or using a nucleotide sequence analyzer such as 373A DNA Sequencer (manufactured by Perkin Elmer) or the like.

Examples of the vector used for the integration of the amplified DNA fragment include pBluescript KS(+) (manufactured by Stratagene), pDIRECT [Nucleic Acids Research, 18, 6069 (1990)], pCR-Script Amp SK(+) [manufactured by Stratagene, Strategies, 5, 6264 (1992)], pT7Blue (manufactured by Novagen), pCR II [manufactured by Invitrogen, Biotechnology, 9, 657 (1991)], pCR-TRAP (manufactured by Genehunter), pNoTA_{T7} (manufactured by 5'→3') and the like.

Novelty of the nucleotide sequence determined in this manner can be verified by searching a data base, such as GenBank, EMBL, DDBJ and the like, using a homology searching program, such as blast and the like, thereby finding that there is no nucleotide sequence which shows an obvious homology that coincides with the nucleotide sequences in the data base.

Examples of the thus obtained partial DNA fragment of cDNA of the IgA nephropathy-related gene include DNA comprising a nucleotide sequence represented by SEQ ID No:41.

When the DNA obtained by the above-described method is a partial DNA fragment of cDNA which corresponds to IgA nephropathy-related mRNA, full length cDNA can be obtained by the following method (1) or (2) using the DNA obtained by the above-described method.

### (1) Application of cDNA library

A full length cDNA can be obtained by carrying out screening according to hybridization using the above-described DNA fragment as the probe and various cDNA libraries.

The method for the preparation of cDNA libraries is described below.

Examples of the method for the preparation of cDNA libraries include methods described in Molecular Cloning, 2nd. ed., Current Protocols in Molecular Biology, or DNA Cloning 1: Core Techniques, A Practical Approach, Second Addition, Oxford University Press (1995), etc. or methods using a commercially available kit, such as Superscript Plasmid System for cDNA Synthesis and Plasmid Cloning (manufactured by Gibco BRL) or ZAP-cDNA Synthesis Kit (manufactured by Stratagene). Additionally, commercially available cDNA libraries, such as a human leukocyte cDNA library (manufactured by Life Technologies) and the like, can also be used.

In preparing the cDNA library, any one of phage vectors, plasmid vectors and the like can be used as the cloning vector which replicates autonomously in *Escherichia coli* K12. Examples include ZAP Express [manufactured by Stratagene, Strategies, 5, 58 (1992)], pBluescript II SK(+) *[*Nucleic Acids Research, 17, 9494 (1989)], λ zap II (manufactured by Stratagene), λgt10, λgt11 [DNA Cloning, A Practical Approach, 1, 49 (1985)], λTriplEx (manufactured by Clontech), λBlueMid (manufactured by Clontech), λExCell (manufactured by Pharmacia), pcD2 [Mol. Cell. Biol., 3, 280 (1983)], pUC18 [Gene, 33, 103 (1985)], and the like.

With regard to the *Escherichia coli* used to transform with the vector containing the cDNA, any microorganism belonging to *Escherichia coli* can be used. Examples include *Escherichia coli* XL1-Blue MRF' [manufactured by Stratagene, Strategies, 5, 81 (1992)], *Escherichia coli* C600 [Genetics, 39, 440 (1954)], *Escherichia coli* Y1088 [Science, 222, 778 (1983)], *Escherichia coli* Y1090 *[*Science, 222, 778 (1983)], *Escherichia coli* NM522 *[*J. Mol. Biol., 166, 1 (1983)], *Escherichia coli* K802 *[*J. Mol. Biol., 16, 118 (1966)], *Escherichia coli* JM105 [Gene, 38, 275 (1985)], and the like.

A cDNA clone can be selected from the cDNA library according to a colony hybridization or plaque hybridization method (*Molecular Cloning,* 2nd ed.) using a probe labeled with an isotope or digoxigenin.

The DNA of interest can be obtained from the thus selected clone in the usual way.
(2) The DNA of interest can also be obtained by the 5'-RACE (rapid amplification of cDNA ends) and 3'-RACE method [Proc. Natl. Acad. Sci. USA, 85, 8998 (1988)] in which cDNA is synthesized from mRNA by the above-described method, adapters are added to both ends of the cDNA and then PCR is carried out using primers based on the nucleotide sequence of the adapter and the nucleotide sequence of the amplified fragment.

The nucleotide sequence of the DNA obtained by these methods can be determined by the above-described nucleotide sequence determining method. Novelty of the sequence can also be verified by the above-described method.

Examples of the novel full length cDNA of the IgA nephropathy-related gene obtained in this manner include DNAs having the nucleotide sequences represented by SEQ ID NO:1 to NO:7.

Once a DNA of IgA nephropathy-related gene is obtained and a nucleotide sequence thereof is determined in the above-described manner, the DNA of interest can be obtained by PCR [PCR Protocols, Academic Press (1990)] by preparing primers based on the nucleotide sequence and using cDNA synthesized from the mRNA or a cDNA library as the template. Alternatively, the DNA of interest may be prepared by chemical synthesis using a DNA synthesizer based on the determined DNA nucleotide sequence. Examples of the DNA synthesizer include DNA Synthesizer Model 392 (manufactured by Perkin-Elmer) using the phosphoramidite method.

On the basis of the nucleotide sequence information of the DNA and DNA fragments isolated according to the above methods, an oligonucleotide having a partial sequence of the IgA nephropathy-related DNA and a corresponding antisense oligonucleotide can be prepared according to the usual methods described in *Molecular Cloning*, 2nd ed. *etc*. or a DNA synthesizer using the nucleotide sequence information of the DNA.

Examples of the oligonucleotide include DNA comprising a sequence which is the same as continuous 5 to 60 nucleotides in a nucleotide sequence of the above DNA, and DNA comprising a sequence complementary to the DNA. Specific examples include DNA comprising continuos 5 to 60 nucleotides in a nucleotide sequence represented by SEQ ID NO:1, and DNA having a sequence complementary to the DNA.

As the sense primer and antisense primer, the above-described oligonucleotides in which melting point (Tₘ) and the number of bases are not significantly different from each other are preferred. Specific examples include oligonucleotides comprising a nucleotide sequence selected from' the nucleotide sequences represented by SEQ ID NO:47 and NO:48.

Also, analogues of these oligonucleotide (hereinafter also referred to as "analogous oligonucleotides") can also be used as the oligonucleotide of the present invention.

Examples of the analogous oligonucleotides include analogous oligonucleotides in which a phosphodiester bond in an oligonucleotide is converted to a phosphorothioate bond, analogous oligonucleotides in which a phosphodiester bond in an oligonucleotide is converted to an N3'-P5' phosphoamidate bond, analogous oligonucleotides in which ribose and a phosphodiester bond in an oligonucleotide is converted to a peptide nucleic acid bond, analogous oligonucleotides in which uracil in an oligonucleotide is replaced with C-propynyluracil, analogous oligonucleotides in which uracil in an oligonucleotide is replaced with C-5 thiazoluracil, analogous oligonucleotides in which cytosine in an oligonucleotide is replaced with C-5 propynylcytosine, analogous oligonucleotides in which cytosine in an oligonucleotide is replaced with phenoxazin-modified cytosine, analogous oligonucleotides in which ribose in an oligonucleotide is replaced with 2'-O-propylribose, analogous oligonucleotides in which ribose in an oligonucleotide is replaced with 2'-methoxyethoxyribose, and the like [Cell Engineering, 16, 1463 (19997)].

### 2. Production of protein having an activity related to IgA nephropathy

The full length cDNA of IgA nephropathy-related gene obtained by the method described in the above section 1 encodes a protein having an activity related to IgA nephropathy (referred to as "IgA nephropathy-related protein" hereinafter).

The IgA nephropathy-related protein of the present invention can be prepared by expressing the IgA nephropathy-related gene in a host cell using the method described in Molecular Cloning, 2nd ed., Current Protocols in Molecular Biology, and the like, for example, according to the following method.

A DNA fragment having a suitable length containing a portion encoding the protein (hereinafter referred to as "IgA nephropathy protein-encoding DNA") is prepared from the full length cDNA as occasion demands.

Also, DNA in which nucleotides in a nucleotide sequence at a part encoding the protein of the present invention are replaced to give a codon suitable for expression of the host cell as occasion demands. The DNA is useful for efficiently producing the protein of the present invention.

An expression plasmid of the protein is prepared by inserting the DNA fragment or the full length cDNA into a downstream site of the promoter in the expression vector.

As the host cell, bacteria, yeasts, animal cells, insect cells, plant cells, and the like can be used so long as they can express the gene of interest.

Examples of the expression vector include those which can replicate autonomously in the above-described host cell or can be integrated into chromosome and have a promoter at such a position that the IgA nephropathy protein-encoding DNA can be transcribed.

When a bacterium or the like is used as the host cell, it is preferred that the IgA nephropathy protein-encoding DNA expression vector can replicate autonomously in the bacterium and is a recombinant vector constructed with a promoter, a ribosome binding sequence, the IgA nephropathy protein-encoding DNA and a transcription termination sequence. A promoter controlling gene can also be contained.

Examples of the expression vector include pBTrp2, pBTac1 and pBTac2 (all available from Boehringer Mannheim Co.), pKK233-2 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene) , pTrs30 [prepared from *Escherichia coli* JM109/pTrS30 (FERM BP-5907)], pTrs32 [prepared from *Escherichia coli* JM109/pTrS32 (FERM BP-5408)], pGHA2 [prepared from *Escherichia coli* IGHA2 (FERM B-400), Japanese Published Unexamined Patent Application No. 221091/85], pGKA2 [*Escherichia* coli IGKA2 (FERM BP-6798), Japanese Published Unexamined Patent Application No. 221091/85], pTerm2 (U.S. Patents 4,686,191, 4,939,094 and 5,160,735), pSupex, pUB110, pTP5, pC194, pEG400 [J. Bacteriol., 172, 2392 (1990)], pGEX (manufactured by Pharmacia), pET system (manufactured by Novagen), and the like.

With regard to the promoter, any promoter can be used so long as it can function in the host cell. Examples include promoters originated from *Escherichia coli*, phage and the like, such as trp promoter (P*trp*) , *lac* promoter (P*lac*), P_{L} promoter, P_{R} promoter, T7 promoter and the like, SPO1 promoter, SPO2 promoter, penP promoter and the like. Also, artificially designed and modified promoters, such as a promoter in which two Ptrp are linked in series (P*trp* × 2), tac promoter, letI promoter [Gene, 44, 29 (1986)] and lacT7 promoter and the like, can be used.

It is preferred to use a plasmid in which the space between Shine-Dalgarno sequence which is the ribosome binding sequence and the initiation codon is adjusted to an appropriate distance (for example, 6 to 18 bases).

The transcription termination sequence is not always necessary for the expression of the IgA nephropathy-related gene of the present invention. However, it is preferred to arrange the transcription terminating sequence at just downstream of the structural gene.

Examples of the host cell include microorganisms belonging to the genus *Escherichia*, *Serratia*, *Bacillus, Brevibacterium*, *Corynebacterium*, *Microbacterium, Pseudomonas,* and the like. Specific examples include *Escherichia* coli XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Escherichia coli* GI698, *Escherichia coli* TB1, *Serratia ficaria, Serratia fonticola, Serratia liquefaciens, Serratia marcescens*, *Bacillus subtilis, Bacillus amyloliquefaciens, Brevibacterium ammoniagenes, Brevibacterium immariophilum* ATCC 14068, *Brevibacterium saccharolyticum* ATCC 14066, *Brevibacterium flavum* ATCC 14067, *Brevibacterium lactofermentum* ATCC 13869, *Corynebacterium glutamicum* ATCC 13032, *Corynebacterium glutamicum* ATCC 13869, *Corynebacterium acetoacidophilum* ATCC 13870, *Microbacterium ammoniaphilum* ATCC 15354, *Pseudomonas putida, Pseudomonas* sp. D-0110 and the like.

With regard to the method for the introduction of the recombinant vector, any method for introducing DNA into the above-described host cells, such as a method in which calcium ion is used [Proc. Natl. Acad. Sci. USA, 69, 2110 (1972)], a protoplast method (Japanese Published Unexamined Patent Application No. 2483942/88), the methods described in Gene, 17, 107 (1982) and Molecular & General Genetics, 168, 111 (1979) and the like, can be used.

When yeast is used as the host cell, examples of expression vector include YEp13 (ATCC 37115), YEp24 (ATCC 37051), YCp50 (ATCC 37419), pHS19, pHS15, and the like.

Any promoter can be used so long as it can drive the expression in yeast. Examples include a promoter of a gene in the glycolytic pathway, such as hexose kinase and the like, PHO5 promoter, PGK promoter, GAP promoter, ADH promoter, gal 1 promoter, gal 10 promoter, a heat shock protein promoter, MF α1 promoter, CUP 1 promoter, and the like.

Examples of the host cell include microorganisms belonging to the genus *Saccharomyces,* the genus *Schizosaccharomyces,* the genus *Kluyveromyces,* the genus *Trichosporon,* the genus *Schwanniomyces,* the genus *Pichia,* the genus *Candida* and the like. Specific examples include *Saccharomyces cerevisiae, Schizosaccharomyces pombe, Kluyveromyces lactis, Trichosporon pullulans, Schwanniomyces alluvius, Candida utilis* and the like.

With regard to the method for the introduction of the recombinant vector, any method for introducing DNA into yeast, such as an electroporation method [Methods. Enzymol., 194, 182 (1990)], a spheroplast method [Proc. Natl. Acad. Sci. USA, 75, 1929 (1978)], a lithium acetate method [J. Bacteriol., 153, 163 (1983)], a method described in Proc. Natl. Acad. Sci. USA, 75, 1929 (1978) and the like, can be used.

When animal cells are used as the host cells, examples of expression vector include pcDNAI and pcDM8 (both available from Funakoshi), pAGE107 [Japanese Published Unexamined Patent Application No. 22979/91; Cytotechnology, 3, 133 (1990)], pAS3-3 (Japanese Published Unexamined Patent Application No. 227075/90), pcDM8 [Nature, 329, 840 (1987)], pcDNAI/Amp (manufactured by Invitrogen), pREP4 (manufactured by Invitrogen), pAGE103 [J. Biochem., 101, 1307 (1987)], pAGE210, and the like.

Any promoter can be used so long as it can function in animal cell. Examples include a promoter of IE (immediate early) gene of cytomegalovirus (CMV), an early promoter of SV40, a promoter of retrovirus, a metallothionein promoter, a heat shock promoter, SRα promoter, and the like. Also, the enhancer of the IE gene of human CMV may be used together with the promoter.

Examples of the host cell include human Namalwa cell, monkey COS cell, Chinese hamster CHO cell, HST5637 (Japanese Published Unexamined Patent Application No. 299/88), and the like.

With regard to the method for the introduction of the recombinant vector into animal cells, any method for introducing DNA into animal cells, such as an electroporation method [Cytotechnology, 3, 133 (1990)], a calcium phosphate method (Japanese Published Unexamined Patent Application No. 227075/90), a lipofection method [Proc. Natl. Acad. Sci. USA, 84, 7413 (1987)] and the method described in Virology, 52, 456 (1973), can be used. Preparation and culturing of transformants can be carried out in accordance with the method described in Japanese Published Unexamined Patent Application No. 227075/90 or Japanese Published Unexamined Patent Application No. 257891/90.

When an insect cell is used as the host cell, the protein can be expressed by known methods described in, for example, Bacurovirus Expression Vectors, A Laboratory Manual, Current Protocols in Molecular Biology, supplement 1-38 (1987-1997) Bio/Technology, 6, 47 (1988), or the like.

That is, a recombinant gene transfer vector and baculovirus are simultaneously inserted into an insect cell to obtain a recombinant virus in an insect cell culture supernatant, and then the insect cells are infected with the thus obtained recombinant virus to effect expression of the protein.

Examples of the gene introducing vector used in the method include pVL1392, pVL1393, pBlueBacIII (all manufactured by Invitrogen), and the like.

Examples of the baculovirus include Autographa californica nuclear polyhedrosis virus with which insects of the family Barathra are infected, and the like.

Examples of the insect cell include *Spodoptera frugiperda* oocytes Sf9 and Sf21 (Bacurovirus Expression Vectors, A Laboratory Manual, W.H. Freeman and Company, New York, (1992)), *Trichoplusia* ni oocyte High 5 (manufactured by Invitrogen) and the like.

The method for the co-transfer of the above-described recombinant gene transfer vector and the above-described baculovirus for the preparation of the recombinant virus include calcium phosphate method (Japanese Published Unexamined Patent Application No. 227075/90), lipofection method [Proc. Natl. Acad. Sci. USA, 84, 7413 (1987)] and the like.

When a plant cell is used as the host cell, examples of expression vector include a Ti plasmid, a tobacco mosaic virus vector, and the like.

Any promoter can be used so long as it can drive the expression in a plant cell. Examples include 35S promoter of cauliflower mosaic virus (CaMV), rice actin 1 promoter, and the like.

Examples of the host cell include plant cells and the like, such as tobacco, potato, tomato, carrot, soybean, rape, alfalfa, rice, wheat, barley, and the like.

With regard to the method for the introduction of the recombinant vector, any method for introducing DNA into plant cells, such as the Agrobacterium method (Japanese Published Unexamined Patent Application No. 140885/84, Japanese Published Unexamined Patent Application No. 70080/85, WO 94/00977), the electroporation method (Japanese Published Unexamined Patent Application No. 251887/85), the particle gun method (Japanese Patents 2606856 and 2517813), and the like.

With regard to the gene expression method, a secretion production, a fusion protein expression and the like can be effected in accordance with the method described in *Molecular Cloning*, 2nd ed., in addition to the direct expression.

When expressed in a yeast, an animal cell or an insect cell, a glycoprotein or glycosylated protein can be obtained.

The IgA nephropathy-related protein can be produced by culturing the transformant of the present invention obtained in the above in a culture medium to produce and accumulate the IgA nephropathy-related protein, and recovering the protein from the resulting culture.

Culturing of the transformant used in the production of the IgA nephropathy-related protein of the present invention in a culture medium is carried out in accordance with a usual method used in culturing of respective host cells.

When the transformant of the present invention is an prokaryote, such as *Escherichia coli* or the like, or an eukaryote, such as yeast or the like, the medium used in culturing of these transformants may be either a natural medium or a synthetic medium, so long as it contains a carbon source, a nitrogen source, an inorganic salt and the like which can be assimilated by the transformants and can perform culturing of the transformant efficiently.

Examples of the carbon source include those which can be assimilated by respective transformants, such as carbohydrates (for example, glucose, fructose, sucrose, molasses containing them, starch, starch hydrolysate, and the like), organic acids (for example, acetic acid, propionic acid, and the like), and alcohols (for example, ethanol, propanol, and the like).

Examples of the nitrogen source include ammonia, various ammonium salts of inorganic acids or organic acids (for example, ammonium chloride, ammonium sulfate, ammonium acetate, ammonium phosphate, and the like), other nitrogen-containing compounds, peptone, meat extract, yeast extract, corn steep liquor, casein hydrolysate, soybean meal and soybean meal hydrolysate, various fermented cells and hydrolysates thereof, and the like.

Examples of inorganic substance used in the culture medium include potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culturing is carried out under aerobic conditions by shaking culture, aeration stirring culture or the like means. The culturing temperature is preferably from 15 to 45°C, and the culturing time is generally from 16 hours to seven days. The pH of the medium is maintained at 3.0 to 9.0 during the culturing. Adjustment of the medium pH is carried out using an inorganic or organic acid, an alkali solution, urea, calcium carbonate, ammonia and the like.

Also, antibiotics (for example, ampicillin, tetracycline, and the like) may be added to the medium during the culturing as occasion demands.

When a microorganism transformed with a recombinant vector containing an inducible promoter is cultured, an inducer may be added to the medium as occasion demands. For example, isopropyl-β-D-thiogalactopyranoside (IPTG) or the like may be added to the medium when a microorganism transformed with a recombinant vector containing lac promoter is cultured, or indoleacrylic acid (IAA) or the like may by added thereto when a microorganism transformed with an expression vector containing *trp* promoter is cultured.

Examples of the medium used in the culturing of a transformant obtained using an animal cell as the host cell include RPMI 1640 medium [The Journal of the American Medical Association, 199, 519 (1967)], Eagle's MEM medium [Science, 122, 501 (1952)], Dulbecco's modified MEM medium [Virology, 8, 396 (1959)], 199 Medium [Proceeding of the Society for the Biological Medicine, 73, 1 (1950)], and any one of these media further supplemented with fetal calf serum.

The culturing is carried out generally at pH of 6 to 8 and at a temperature of 30 to 40°C for a period of 1 to 7 days in the presence of 5% CO₂.

As occasion demands, antibiotics (for example, kanamycin, penicillin, and the like) may be added to the medium during the culturing.

Examples of the medium used in the culturing of a transformant obtained using an insect cell as the host cell, include TNM-FH medium (manufactured by Pharmingen), Sf-900 II SFM (manufactured by Life Technologies), ExCell 400 or ExCell 405 (both manufactured by JRH Biosciences), Grace's Insect Medium [Nature, 195, 788 (1962)], and the like.

The culturing is carried out generally at pH of 6 to 7 and at a temperature of 25 to 30°C for a period of 1 to 5 days.

Additionally, antibiotics (for example, gentamicin, and the like) may be added to the medium during the culturing as occasion demands.

A transformant obtained by using a plant cell as the host cell can be used as the cell or after differentiating a plant cell or organ. Examples of the medium used in the culturing of the transformant include Murashige and Skoog (MS) medium, White medium, media to which a plant hormone, such as auxin, cytokinine, or the like has been added, and the like.

The culturing is carried out generally at pH of 5 to 9 and at a temperature of 20 to 40°C for a period of 3 to 60 days.

Additionally, antibiotics (for example, kanamycin, hygromycin and the like) may be added to the medium during the culturing as occasion demands.

As described above, the protein can be produced by culturing a transformant derived from a microorganism, animal cell or plant cell containing a recombinant vector to which a DNA encoding the protein of the present invention has been inserted according to the general culturing method to produce and accumulate the protein, and recovering the protein from the resulting culture.

With regard to the gene expression method, a secretion production, a fusion protein expression and the like can be effected in accordance with the method described in *Molecular Cloning,* 2nd ed., in addition to the direct expression.

The method for producing the protein of the present invention includes a method of intracellular expression in a host cell, a method of extracellular secretion from a host cell, or a method of production on a host cell membrane outer envelope. The method can be selected by changing the used host cell or the structure of the produced protein.

When the protein of the present invention is produced in a host cell or on a host cell membrane outer envelope, the protein can be positively secreted extracellularly according to the method of Paulson et al. [J. Biol. Chem. , 264, 17619 (1989)], the method of Lowe et al. [Proc. of Natl. Acad. Sci. USA, 86, 8227 (1989); Genes Develop., 4, 1288 (1990)], and the methods described in Japanese Published Unexamined Patent Application No. 336963/93, Japanese Published Unexamined Patent Application No. 823021/94 (WO94/23021), and the like.

Specifically, the protein of the present invention can be positively secreted extracellularly by expressing it in the form that a signal peptide has been added to the foreground of a protein containing an active site of the protein of the present invention according to the recombinant DNA technique.

Furthermore, the production amount can be increased by utilizing a gene amplification system using a dihydrofolate reductase gene according to the method described in Japanese Published Unexamined Patent Application No. 227075/90.

Moreover, the protein of the present invention can be produced by redifferentiating a transgenic animal or plant cell to develop a transgenic animal individual (transgenic nonhuman animal) or plant individual (transgenic plant) and using the individual.

When the transformant is the animal individual or plant individual, the protein of the present invention can be produced by breeding or cultivating it to produce and accumulate the protein, and recovering the protein from the animal individual or plant individual.

Examples of the method for producing the protein of the present invention using the animal individual include a method for producing the protein of the present invention in an animal developed by inserting a gene according to a known method [American Journal of Clinical Nutrition, 63, 639S (1996), American Journal of Clinical Nutrition, 63, 627S (1996), Bio/Technology, 9, 830 (1991)].

In the animal individual, the protein can be produced by breeding a transgenic nonhuman animal to which the DNA encoding the protein of the present invention has been inserted to produce and accumulate the protein in the animal, and recovering the protein from the animal. Examples of the production and accumulation place in the animal include milk (Japanese Published Unexamined Patent Application No. 309192/88), egg and the like of the animal. Any promoter can be used, so long as it can drive the expression in the animal. Suitable examples include an α-casein promoter, a β-casein promoter, a β-lactoglobulin promoter, a whey acidic protein promoter, and the like, which are specific for mammary glandular cells.

Examples of the method for producing the protein of the present invention using the plant individual include a method for producing the protein of the present invention by cultivating a transgenic plant to which the DNA encoding the protein of the present invention by a known method [Tissue Culture, 20 (1994), Tissue Culture, 21 (1994), Trends in Biotechnology, 15, 45 (1997)] to produce and accumulate the protein in the plant, and recovering the protein from the plant.

When the protein of the present invention having an activity related to IgA nephropathy is isolated and purified from a culture of the transformant of the present invention, usual methods for the isolation and purification of enzymes may be used.

For example, when the protein of the present invention is expressed in a dissolved state inside the cells, the cells after completion of the culturing are recovered by centrifugation, suspended in a buffer of aqueous system and then disrupted using ultrasonic oscillator, French press, Manton Gaulin homogenizer, dynomill or the like to obtain a cell-free extract. A purified product can be obtained from a supernatant fluid prepared by centrifugation of the cell-free extract, by employing a technique or a combination of techniques, such as solvent extraction, salting out with ammonium sulfate or the like, desalting, precipitation with organic solvents, anion exchange chromatography using a resin [for example, diethylaminoethyl (DEAE)-Sepharose, DIAION HPA-75 (manufactured by Mitsubishi Chemical), or the like], cation exchange chromatography using a resin [for example, S-Sepharose FF (manufactured by Pharmacia), or the like], hydrophobic chromatography using a resin (for example, butyl-Sepharose, phenyl-Sepharose, or the like), gel filtration using a molecular sieve, affinity chromatography, chromatofocusing, electrophoresis (for example, isoelectric focusing).

Also, when the protein is expressed inside the cells in the form of an inclusion body, the cells are recovered, disrupted and centrifuged to thereby recover the inclusion body of the protein as a precipitated fraction.

The recovered inclusion body of the protein is solubilized using a protein denaturing agent.

The protein is renatured into a normal solid structure by diluting or dialyzing the thus-obtained solubilized solution to lower the protein denaturing agent in the solubilized solution. After this operation, a purified product of the protein is obtained by the isolation and purification method in the same manner as described above.

When the protein of the present invention or a derivative thereof, such as a sugar-modified product, is secreted extracellularly, the protein or the derivative can be recovered from the culture supernatant. That is, the purified product can be obtained by recovering culture supernatant from the culture by a technique, such as centrifugation or the like, and then subjecting the culture supernatant to the above-described isolation and purification method.

Examples of the protein obtained in this manner include proteins having an amino acid sequence selected from the amino acid sequences represented by SEQ ID NO:34 to NO:40.

Additionally, the protein expressed by the above-described method can be produced by a chemical synthesis method, such as Fmoc method (fluorenylmethyloxycarbonyl method), tBoc method (t-butyloxycarbonyl method) or the like. It can also be synthesized using a peptide synthesizer available from Sowa Boeki (manufactured by Advanced ChemTech, USA), Perkin-Elmer Japan (manufactured by Perkin-Elmer, USA), Pharmacia Biotech (manufactured by Pharmacia Biotech, Sweden), Aroka (manufactured by Protein Technology Instrument, USA), KURABO (manufactured by Synthecell-Vega, USA), Japan PerSeptive Limited (manufactured by PerSeptive, USA) or Shimadzu Corporation or the like.

### 3. Preparation of antibody which recognizes the protein of the present invention

### (1) Preparation of antigen

A purified product of a full length or a partial fragment of the protein obtained by the method described in the above section 2 or a peptide having a partial amino acid sequence of the protein of the present invention is used as the antigen.

A partial protein sequence having 5 to 30 residues is selected as the partial peptide for the antigen. In order to obtain an antibody which recognizes the protein having a non-denatured natural structure, a partial sequence stereostructurally present on the surface of the protein is preferably selected as the antigen peptide. The part stereostructurally present on the surface of the protein can be supposed by expecting a partial sequence having high hydrophilicity according to the method of Kyte and Doolittle [Journal of Molecular Biology, 157, 105-132 (1982)] and the like. Specifically, generally, a part having a low hydrophilicity is often present in the inside of the protein stereostructurally, whereas a part having a high hydrophilicity is present on the surface of the protein. Furthermore, the N-terminal and C-terminal of the protein are often present on the surface of the protein. However, the thus selected peptide is not always used as an antigen for the objective antibody.

When the partial peptide is used as the antigen, cysteine can be added to the terminal so that the peptide can be crosslinked with other protein. When the sequence inside of the protein is selected, the N-terminal and C-terminal are optionally acetylated and amidated, respectively.

The partial peptide can be synthesized by liquid-phase and solid-phase peptide synthesis methods, a method in which they are suitably combined, or a method following them [International Journal of Peptide Protein Research, 35, 161-214 (1990), Methods in Enzymology, 289, (1997), Methods in Molecular Biology, 35, (1994)].

Moreover, an automatic peptide synthesizer can also be used. Synthesis using the automatic peptide synthesizer can be carried out by using N^{α}-Fmoc amino acid or N^{α}-Boc amino acid of which side chain has been suitably protected etc. with a commercially available peptide synthesizer, such as a peptide synthesizer manufactured by Shimadzu Corporation, a peptide synthesizer manufactured by Advanced ChemTech Inc., USA (hereinafter referred to as "ACT Co."), or the like, according to a respective synthesis program. A protecting amino acid and a carrier resin as starting materials can be obtained from ABI Co., Shimadzu Corporation, Kokusan Kagaku Co., Ltd., NovaBiochem, ACT Co., AnaSpec Inc., Peptide Institute. Inc., or the like.

### (2) Immunization of animal and preparation of antibody

### (i) Production of polyclonal antibody

A polyclonal antibody can be produced by administering the antigen obtained by the above method to an animal.

Examples of the animal to be administered include rabbits, goats, rats, mice, hamsters and the like.

Preferable dosage of antigen is 50 to 100 µg per animal.

The immunization is carried out by administering to an animal the antigen through subcutaneous, intravenous or intraperitoneal injection together with an appropriate adjuvant (for example, complete Freund's adjuvant, aluminum hydroxide gel, pertussis vaccine, or the like). When the antigen is a partial peptide, a conjugate with a carrier protein, such as BSA (bovine serum albumin), KLH (keyhole limpet hemocyanin), or the like, is produced, and it is used as the immunogen.

The administration of the antigen is carried out 3 to 10 times at the intervals of 1 or 2 weeks after the first administration. On the 3rd to 7th day after each administration, a blood sample is collected from the venous plexus of the eyeground, and it is confirmed that the serum reacts with the antigen by the enzyme immunoassay [Antibodies - A Laboratory Manual, Cold Spring Harbor Laboratory (1988)] or the like.

Serum is obtained from a non-human mammal having an enough antibody titer against the antigen used for the immunization, and the serum is isolated and purified to obtain a polyclonal antibody.

With regard to the method for the isolation and purification, centrifugation, salting out by 40-50% ammonium sulfate, caprylic acid precipitation [Antibodies, A Laboratory manual, Cold Spring Harbor Laboratory (1988)], or chromatography using a DEAE-Sepharose column, an anion exchange column, a protein A or G-column, a gel filtration column and the like may be employed alone or as a combination thereof.

### (ii) Production of monoclonal antibody

A monoclonal antibody can be prepared by preparing a hybridoma through fusion of the antibody-producing cells with myeloma cells of a non-human mammal and culturing the hybridoma, or administering the hybridoma to an animal to induce ascites tumor in the animal, and then isolating and purifying it from the culture medium or ascitic fluid.

Examples of the antibody-producing cells include spleen cells, lymph nodes and antibody-producing cells in peripheral blood. Particularly, spleen cells are preferred.

A method for producing a monoclonal antibody using spleen cells is described below; however, other antibody-producing cells can also be carried out in the same manner.

The spleen is excised from rats showing an antibody titer 3 to 7 days after the final immunization.

The spleen is cut to pieces in MEM medium (manufactured by Nissui Pharmaceutical), loosened using a pair of forceps, followed by centrifugation at 1,200 rpm for 5 minutes, and the resulting supernatant is discarded.

The spleen in the precipitated fraction is treated with a Tris-ammonium chloride buffer (pH 7.65) for 1 to 2 minutes to eliminate erythrocytes and washed three times with MEM medium, and the resulting spleen cells were used as antibody-producing cells.

### (b) Preparation of myeloma cells

As myeloma cells, an established cell line obtained from mouse or rat is used. Examples include cell lines derived from mouse, such as P3-X63Ag8-U1 (hereinafter referred to as "P3-U1") [Current Topics in Microbiology and Immunology, 81, 1 (1978), European J. Immunology, 6, 511 (1976)], SP2/O-Ag14 (SP-2) [Nature, 276, 269 (1978)], P3-X63-Ag8653 (653) *[*J. Immunology, 123, 1548 (1979)], P3-X63-Ag8 (X63) cell line [Nature, 256, 495 (1975)] and the like, which are 8-azaguanine-resistant mouse (BALB/c) myeloma cell lines. These cell lines were subcultured in 8-azaguanine medium [medium in which, to a medium obtained by adding glutamine (1.5 mM), 2-mercaptoethanol (5×10⁻⁵ M), gentamicin (10 µg/ml) and fetal calf serum (FCS) (manufactured by CSL, 10%) to RPMI-1640 medium (hereinafter referred to as the "normal medium"), 8-azaguanine has been further added] and cultured in the normal medium 3 or 4 days before cell fusion, and 2×10⁷ or more of the cells are used for the fusion.

### (c) Production of hybridoma

The antibody-producing cells obtained in (a) and the myeloma cells obtained in (b) are washed with MEM medium or PBS (disodium hydrogen phosphate: 1.83 g, sodium dihydrogen phosphate: 0.21 g, sodium chloride: 7.65 g, distilled water: 1 liter, pH: 7.2) and mixed to give a ratio of antibody-producing cells : myeloma cells = 5 to 10 : 1, followed by centrifugation at 1,200 rpm for 5 minutes, and the supernatant is discarded.

The cells in the resulting precipitated fraction were thoroughly loosened, 0.2 to 1 ml of a mix solution of 2 g polyethylene glycol-1000 (PEG-1000), 2 ml MEM medium and 0.7 ml dimethylsulfoxide (DMSO) per 10⁸ antibody-producing cells is added to the cells under stirring at 37°C, and then 1 to 2 ml of MEM medium is further added thereto several times at 1 to 2 minute intervals.

After the addition, MEM medium is added to give a total amount of 50 ml. The resulting prepared solution is centrifuged at 900 rpm for 5 minutes, and then the supernatant is discarded. The cells in the resulting precipitated fraction were gently loosened and then gently suspended in 100 ml of HAT medium [the normal medium to which hypoxanthine (10⁻⁴ M), thymidine (1.5x10⁻⁵ M) and aminopterin (4×10⁻⁷ M) have been added] by repeated drawing up into and discharging from a measuring pipette.

The suspension is poured into a 96 well culture plate at 100 µl/well and cultured at 37°C for 7 to 14 days in an atmosphere of 5% CO₂ in a 5% CO₂ incubator.

After culturing, a part of the culture supernatant is recovered, and a hybridoma which specifically reacts with a partial fragment protein of the protein of the present invention is selected according to the enzyme immunoassay described in Antibodies, A Laboratory manual, Cold Spring Harbor Laboratory, Chapter 14 (1998) and the like.

A specific example of the enzyme immunoassay is described below.

A 1 to 50 µg/ml portion of the antigen used in the immunization is poured into a 96 well plate for EIA at 10 to 100 µl/hole, and allowed to stand at 4°C overnight for coating on the plate.

After allowing to stand, PBS solution containing 1% BSA (hereinafter referred to as "BSA-PBS") at 100 to 200 µl/hole and allowed to stand at room temperature for 1 to 2 hours or at 4°C for one to two nights for blocking protein-binding residues which remain on the plate.

After blocking, BSA-PBS is discarded, and the plate is washed sufficiently with PBS.

The immunized animal serum, polyclonal antibody obtained by purifying from the antiserum, hybridoma culturing supernatant or purified antibody obtained in (d) described below is used as a first antibody, and 1 to 10 µg/ml of the antibody is poured to the plate at 20 to 100 µl/hole, and allowed to stand at room temperature for 2 to 3 hours or at 4°C overnight.

After washing the plate sufficiently with PBS or PBS-0.05% Tween, 1 to 50 µg/ml of an anti-immunoglobulin antibody labeled with a biotin, a chemical luminous substance, a radiation substance or the like as a second antibody is poured at 50 to 100 µl/hole and allowed to react at room temperature for 1 to 2 hours.

After washing the plate sufficiently with PBS-Tween, a reaction is carried out according to the labeled substance of the second antibody.

A hybridoma which specifically reacts with the protein of the present invention in the reaction is selected as one capable of producing a monoclonal antibody of the present invention.

Cloning is repeated using the hybridoma twice by limiting dilution analysis [HT medium (a medium in which aminopterin has been removed from HAT medium) is firstly used, and the normal medium is secondly used], and a hybridoma which shows stable and an enough antibody titer is selected as a hybridoma capable of producing a monoclonal antibody of the present invention.

### (d) Preparation of monoclonal antibody

The monoclonal antibody-producing hybridoma cells obtained in (c) are injected intraperitoneally into 8- to 10-week-old mice or nude mice treated with pristane [intraperitoneal administration of 0.5 ml of 2,6,10,14-tetramethylpentadecane (pristane), followed by 2 weeks of feeding] at 5 to 20×10⁶ cells/animal. The hybridoma causes ascites tumor in 10 to 21 days.

The ascitic fluid is collected from the mice or nude mice, and centrifuged to remove solid contents.

A monoclonal antibody can be purified and isolated from the resulting supernatant according to the method similar to that used in the polyclonal antibody.

The subclass of the antibody can be determined using a mouse monoclonal antibody typing kit or a rat monoclonal antibody typing kit. The protein amount can be determined by the Lowry method or by calculation based on the absorbance at 280 nm.

### 4. Application of IgA nephropathy-related DNA, protein or antibody

(1) Using the DNA described in the above section 1, mRNA of the IgA nephropathy-related gene of the present invention can be detected by northern hybridization (Molecular Cloning, 2nd ed.), PCR [PCR Protocols, Academic Press (1990)], RT (reverse-transcribed)-PCR and the like. Particularly, RT-PCR is simple and easy and can therefore be applied to the diagnosis of IgA nephropathy.

For example, diagnosis of IgA nephropathy may be effected by carrying out PCR using the DNA described in the above section 1 which corresponds to the mRNA to be detected as a pair of oligonucleotide primers and detecting the amplified fragment. In that case, the nucleotide sequence moiety to be amplified may be any nucleotide sequence region of the mRNA, but a nucleotide sequence region which has a length of from 50 bp to 2 kbp and does not contain a repeating sequence or GC (guanine-cytosine) bases-rich sequence is preferred. (2) Using the antisense oligonucleotide (RNA/DNA) described in the above section 1 *[*Chemistry, 46, 681 (1991), Biotechnology, 9, 358 (1992)], treatment of IgA nephropathy can be effected by inhibiting transcription of DNA or translation of mRNA.

An example of the antisense oligonucleotide (RNA/DNA) of the above section 1 used in this case is an antisense oligonucleotide which has a partial nucleotide sequence, preferably a sequence of from 10 to 50 bases in the translation initiation region, of a DNA which encodes the protein described in the above section 2.
(3) Using the DNA described in the above section 1, the IgA nephropathy-related protein of the present invention can be obtained by the method described in the above section 2.
(4) Using the protein described in the above section 2 as the antigen, antibodies can be produced by the method described in the above section 3.
(5) Using the antibody described in the above section 3, the IgA nephropathy-related protein can be detected or determined immunologically.

Examples of the immunological detection method include ELISA method using a microtiter plate, fluorescent antibody technique, western blot technique, immunohistochemical staining and the like.

Examples of the immunological determination method include sandwich ELISA method in which, among antibodies which react with the protein of the present invention in solution, two monoclonal antibodies having different epitopes are used and radioimmunoassay method in which the protein of the present invention labeled with radioactive isotope, such as ¹²⁵I or the like, and an antibody which recognizes the protein of the present invention are used.
(6) Using the antibody described in the above section 3, the presence or absence of IgA nephropathy in a person to be inspected can be diagnosed by immunologically detecting or determining an IgA nephropathy-related protein in leukocytes collected from a healthy person and the person to be inspected, comparing its amounts in the healthy person and person to be inspected and then examining the quantitative fluctuation. As a specific sample to be tested, leukocytes separated from peripheral blood samples of a healthy person and a person to be inspected can be used. Additionally, when the IgA nephropathy-related protein to be detected is a protein secreted from leukocytes, the presence or absence of IgA nephropathy in a person to be inspected can be detected and diagnosed by immunologically detecting or determining the protein in blood plasma samples collected from a healthy person and the person to be inspected, comparing its amounts in the healthy person and person to be inspected and then examining its quantitative fluctuation.
(7) The antibody described in the above section 3 can be applied to the treatment or prevention of IgA nephropathy.

When the DNA, protein and antibody of the present invention is used for the diagnosis, treatment or prevention of IgA nephropathy, a diagnostically or pharmacologically acceptable carrier may be added.

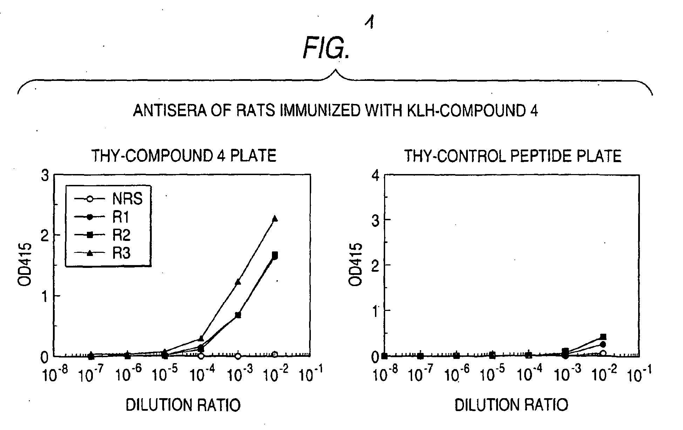

Fig.1 is a graph showing the binding reactivity of rat antisera, which were obtained by immunizing rats with a conjugate of a peptide (Compound 4) having the amino acid sequence represented by SEQ ID NO:117 with KLH, to Compound 4 examined by the enzyme immunoassay. The binding reactivity to Compound 4 is shown in the left, while the binding reactivity to a control peptide is shown in the right. R1 to R3 and NRS show antisera of 3 rats immunized with Compound 4 and a normal rat serum, respectively.

Examples of the present invention are given below by way of illustration and not by way of limitation.

### Example 1: Differential display of leukocytes of IgA nephropathy patients and healthy persons

### (1) Preparation of total RNA from leukocytes of IgA nephropathy patients and healthy persons

A 20 ml portion of blood was collected from each of five IgA nephropathy patients and five healthy persons.

This was mixed with 500 µl of 1,000 units/ml heparin solution to inhibit coagulation, transferred into a centrifugation tube and then centrifuged at 3,300 rpm for 15 minutes at room temperature, and the resulting intermediate layer buffy coat containing leukocytes was transferred into another centrifugation tube.

Thereafter, total RNAs were obtained in accordance with the AGPC method [Experimental Medicine, 9, 1937 (1991)] or using an RNA recovering kit, RNAeasy (manufactured by QIAGEN).

### (2) Fluorescence differential display using leukocyte total RNAs of IgA-nephropathy patients and healthy persons

Distilled water was added to 2.5 µg of each of the total RNAs obtained in the above step (1) to a total volume of 9 µl, and the solution was mixed with 1 µl of an anchor primer (50 µM, custom-synthesized by Sawady Technology) whose 5'-end had been fluorescence-labeled with fluorescein isothiocyanate (referred to as "FITC" hereinafter), heated at 70°C for 5 minutes and then immediately cooled on an ice bath.

Since each of the three primers FAH (nucleotide sequence is shown in SEQ ID NO:107), FGH (nucleotide sequence is shown in SEQ ID NO:108) and FCH (nucleotide sequence is shown in SEQ ID NO:109) was used in each reaction as the 5'-end fluorescence-labeled anchor primer, a total of three combinations of reactions were carried out for one sample of total RNAs.

A 4 µl portion of 5 × reverse transcriptase reaction buffer [250 mM tris(hydroxymethyl)aminomethane (Tris)-HCl (pH 8.3) , 375 mM KCl, 15 mM MgCl₂] was mixed with 2 µl of 100 mM dithiothreitol (DTT), 1 µl of 10 mM dNTP (dATP, dGTP, dTTP and dCTP) , 1 µl of distilled water and 1 µl (200 units) of a reverse transcriptase SUPERSCRIPT II RNase H- Reverse Transcriptase (manufactured by Life Technologies), and the resulting mixture was allowed to stand at room temperature for 10 minutes, allowed to react at 42°C for 50 minutes to synthesize a DNA, and then heated at 90°C for 5. minutes to terminate the reaction.

After the reaction, to the reaction solution was added 40 µl of TE buffer [10 mM Tris-HCl (pH 8.0), 1 mM disodium ethylenediaminetetraacetate (EDTA) (pH 8.0)].

Next, 14.7 µl of distilled water, 2 µl of 10 × PCR buffer [100 mM Tris-HCl (pH 8.8) , 500 mM KCl , 15 mM MgCl₂, 1% Triton X-100] , 0.8 µl of 2.5 mM dNTP, 0.3 µl of 50 µM fluorescence-labeled anchor primer (the same among FAH, FGH and FCH used in the cDNA synthesis) , 1 µl of 10 µM arbitrary primer (manufactured by Operon) and 0.2 µl of Gene Taq DNA polymerase (5 units/µl, manufactured by Nippon Gene) were added to 1 µl of each of the thus synthesized cDNA samples, and the resulting mixture was arranged in Thermal Cycler to carry out PCR.

The PCR was effected by carrying out the reaction at 94°C for 3 minutes, 40°C for 5 minutes and 72°C for 5 minutes, subsequently carrying out a total of 27 cycles of the reaction in which one cycle was composed of the steps of 95°C for 15 seconds, 40°C for 2 minutes and 72°C for 1 minute, and finally carrying out 5 minutes of the reaction at 72°C.

Since each reaction was carried out by a combination of one of the above-described three types as the fluorescence-labeled anchor primer with one of 60 types of OPD-1 to 20, OPE-1 to 20 and OPV-1 to 20 manufactured by Operon Technologies as the arbitrary primer, a total of 180 reactions, and since a reaction of the fluorescence-labeled anchor primer FGH with an arbitrary primer OPB-2 (manufactured by Operon Technologies) was also carried out, a total of 181 reactions were carried out for each of the total RNAs.

A 4 µl portion of each of the PCR reaction solutions was mixed with 3 µl of electrophoresis sample buffer use (95% formamide, 0.1% xylene cyanol, 0.1% Bromophenol Blue), and the mixture was heated at 95°C for 2 minutes, immediately cooled thereafter on an ice bath and then subjected to 2.5 hours or 6% acrylamide gel electrophoresis at 1,500 V. A solution composed of 89 mM Tris, 89 mM boric acid and 2 mM EDTA was used as the electrophoresis buffer. By measuring - fluorescence of the gel after electrophoresis using Fluor Imager (manufactured by Molecular Dynamics), the fragments amplified by PCR were detected and compared. In comparison with 5 cases of the healthy persons, a band which significantly increased or decreased in leukocytes of 5 cases of the IgA nephropathy patients was recorded.

Total RNAs were prepared from other 3 cases of IgA nephropathy patients and 3 cases of healthy persons in the same manner as described in the above step (1) to carry out the differential display of the step (2).

A total of 197 bands which showed increased or decreased fluorescence in both of the above two trials of the differential display were cut off from the gels.

38 µl of distilled water, 5 µl of 10 × PCR buffer, 4 µl of 2.5 mM dNTP, 0.6 µl of an anchor primer (no fluorescence labeling: 34 µM, custom-synthesized by Sawady Technology), 2 µl of 10 µM arbitrary primer and 0.5 µl of Gene Taq DNA polymerase were added to about 1/4 portion of each of the gels thus cut off, the resulting mixture was heated at 94°C for 3 minutes and then a total of 30 cycles of the reaction was carried out in which one cycle was comprised of the steps of 95°C for 15 seconds, 40°C for 2 minutes and 72°C for 1 minute, subsequently carrying out 5 minutes of the reaction at 72°C to complete, PCR₇

Each of the resulting reaction solutions was extracted with phenol-chloroform (1:1) and then with chloroform-isoamyl alcohol (24:1), subsequently carrying out ethanol precipitation.

The thus obtained precipitate (amplified DNA fragments) was dissolved in TE buffer and subjected to 1.5% low melting point agarose gel (SEA PLAQUE GTG, manufactured by FMC Bioproducts) electrophoresis.

After the electrophoresis, the resulting gels were stained with ethidium bromide and then the bands containing amplified fragments were cut off.

The gel was heated at 65°C for 15 minutes to melt - agarose and then extracted with phenol-chloroform and then with chloroform-isoamyl alcohol.

The thus obtained extract was subjected to ethanol precipitation and the resulting precipitate (amplified fragments) was dissolved in 10 µl of TE buffer.

A 1 µl portion of each of the amplified fragments was mixed with 1 µl of a vector for PCR fragment cloning use, pT7BlueT-Vector (manufactured by Novagen), and the amplified fragment was cloned into the plasmid using DNA Ligation Kit ver.1 (manufactured by Takara Shuzo) in accordance with the manual attached to the kit.

Using the thus obtained recombinant plasmid, *Escherichia coli .* DH5α (manufactured by Gibco BRL) was transformed in accordance with a known method, and the resulting transformant was spread on LB agar medium containing 50 µg/ml of ampicillin and cultured overnight at 37°C .

The thus grown ampicillin-resistant transformant was suspended in 20 µl of distilled water, the suspension was mixed with 2.5 µl of 10 × PCR buffer, 2 µl of 2.5 mM dNTP, 0.3 µl of 34 µM anchor primer, 1 µl of 10 µM arbitrary primer and 0.5 µl of a Gene Taq DNA polymerase, and the mixture was subjected to PCR under the same conditions of the above-described re-amplification of amplified fragments and then analyzed by electrophoresis which recognized that an amplified fragment has the same length as in the first differential display.

Nucleotide sequence of the amplified fragment was determined using DNA Sequencer (manufactured by Perkin Elmer). In carrying out the nucleotide sequence determination, Dye Primer Cycle Sequencing Kit manufactured by Perkin Elmer and the method described in the manual attached to the kit were used.

Using restriction enzymes capable of cleaving restriction enzyme sites in the determined nucleotide sequence, the reaction product obtained by the above-described differential display was cleaved and then subjected to electrophoresis to recognize that the position of electrophoresis band corresponding to the thus cut off amplified fragment was changed.

Each of the thus obtained nucleotide sequences was compared with a nucleotide sequence data base GenBank to select a total of 66 clones which were not present among the known nucleotide sequences in the data base or coincided only with the expressed sequence tag among nucleotide sequences in the data base.

### Example 2: Detection of specificity of mRNA expression by RT-PCR.

Using 2 µg of each of the total RNAs obtained in Example 1 from leukocytes of five cases of IgA nephropathy patients and 5 cases of healthy persons, a single-stranded cDNA was synthesized using a single-stranded cDNA synthesis kit, Superscript Preamplification System (manufactured by Life Technologies) in accordance with the method described in the manual attached to the kit.

A 21 µl portion of the thus obtained solution containing the single-stranded cDNA was adjusted to a total volume of 420 µl by adding distilled water.

Using 10 µl portion of the thus prepared solution, the expression level of mRNA-corresponding to each amplified fragment was detected by carrying out RT-PCR in the following manner.

That is, 10 µl of the leukocyte single-stranded cDNA solution was mixed with 15.8 µl of distilled water, 4 µl of 10 x PCR buffer, 3.2 µl of 2.5 mM dNTP, 2 µl of DMSO, 2 µl of 10 µM gene-specific 5'-end side sense primer, 2 µl of 10 µM gene-specific 3' side antisense primer and 2 µl of Gene Taq DNA polymerase which had been diluted to 1 unit/µl, and the resulting mixture was heated at 97°C for 5 minutes, cooled on an ice bath for 5 minutes and then a total of 28 cycles of PCR was carried out in which one cycle was comprised of the steps of 94°C for 30 seconds, 65°C for 1 minute and 72°C for 2 minutes.

After completion of the PCR, 2% agarose gel electrophoresis was carried out, the resulting' gel was stained with 0.01% Cyber Green (manufactured by Takara Shuzo), and the amount of the thus stained amplified fragment was determined by Fluor Imager and used as relative expression quantity of mRNA.

In order to make a correction of the amount of mRNA, the same reaction was carried out on a house keeping gene, glyceraldehyde 3-phosphate dehydrogenase (G3PDH) gene, using specific primers (SEQ ID NO:112 and NO:113) and the expression level of mRNA for each gene was corrected based on the ratio of the expression level of G3PDH mRNA, and then the average value of five cases of IgA nephropathy patients and the average value of 5 cases of healthy persons were compared and 30 gene clones having a difference in their values were selected as genes whose expression quantity was changed in patients with IgA nephropathy. The thus selected genes are summarized in Tables 1-1 and 1-2.

**TABLE 1-1**

| No | Gene | Amplification primer¹⁾ | bp²⁾ | Expression fluctuation | RT-PCR primer⁴⁾ | SEQ **ID** NO.⁵⁾ | RT-PCR cycle number |
|---|---|---|---|---|---|---|---|
| 2 | INP303A | FAH/OPD-5 | 305 | 9.9 | 47, 48 | 41 | 28 |

| | | | | | | | |
|---|---|---|---|---|---|---|---|
| 1): A combination of the anchor primer with the arbitrary primer used in the differential display is shown. 2): The length of the amplified fragment of the differential display is shown. 3): Expression fluctuation is shown as the value of "the average value of mRNA expression levels in 5 cases of IgA nephropathy patients/the average value of mRNA expression levels in 5 cases of healthy persons". 4): The primer used in the RT-PCR is shown by the SEQ ID NO. 5): SEQ ID NO. of the Sequence Listing corresponding to the nucleotide sequence of amplified fragment obtained by the differential display described in Example 1 is shown. | | | | | | | |

Thus, it becomes possible to carry out diagnosis of IgA nephropathy by observing the expression levels of these genes in the leukocytes samples to be tested by PT-PCR using primers of these genes and mRNAs of the samples.

### Example 3: Cloning of full length cDNA and analysis of each cDNA clone

### (1) Cloning of full length cDNA

Cloning of a cDNA containing the nucleotide sequence of amplified fragment obtained by differential display was carried out by optionally using a gene trapper method, plaque hybridization of a cDNA library and a 5'-RACE method. The methods are described below.

### (A) Gene trapper method

A cDNA clone was obtained from a human leukocyte cDNA library (manufactured by Life Technologies) by the following method in which pCMV-SPORT (manufactured by Life Technologies) was used as the vector, using GENE TRAPPER cDNA Positive Selection System (manufactured by Life Technologies).

That is, clones in the cDNA library were made into single-stranded DNA (correspond to the antisense strand of cDNA) using Gene II protein and exonuclease III, and hybridization was carried out using a probe, namely a biotinated oligonucleotide specific for each gene (the 5'-end sense primer specific to each gene, used in the RT-PCR in Example 2, was used).

By allowing the biotinated probe to bind to magnetic beads to which streptoavidin had been immobilized, the above-described single-stranded cDNA hybridized with the probe was isolated.

The single-stranded cDNA clone was released from the probe, made into double-stranded DNA using a DNA polymerase and then *Escherichia coli* was transformed with the double-stranded DNA to obtain a transformant containing the cDNA clone.

Illustrative method employed was as described in the manual attached to the kit.

Each of the thus obtained transformants was suspended in 18 µl of distilled water, the suspension was mixed with 2.5 µl of 10 × PCR buffer, 2 µl of 2.5 mM dNTP, 1 µl of 10 µM gene-specific 5' side sense primer, 1 µl of 10 µM gene-specific 3' side antisense primer and 0.5 µl of Gene Taq DNA polymerase, and the resulting mixture was subjected to PCR under the same conditions as the RT-PCR, subsequently carrying out electrophoresis to isolate a transformant as the cDNA clone of interest in which a fragment having a length deduced from the positions of primers was amplified.

### (B) Screening of cDNA library

Screening of cDNA clones was carried out by means of plaque hybridization using a cDNA library of leukocytes of patient with IgA nephropathy and a cDNA library of a neuroblastoma cell line NB-1.

Prior to the plaque hybridization of each library, PCR was carried out in the same manner as in Example 2, using each cDNA library as the template and using each of the gene-specific RT-PCR primers used in Example 2, and a library, in which a fragment having a length deduced from the position of the primer was amplified, was selected as the library that contains the cDNA clone of the gene of interest.

Using the library, DNAs in plaques were blotted on a nylon membrane Hybond N⁺ (manufactured by Amersham).

Using a plasmid which contained the amplified fragment of each gene and was obtained by the differential display of Example 1, as the template, and each of the gene-specific primers used for the RT-PCR in Example 2 as a primer, PCR was carried out by adding PCR DIG labeling mix (manufactured by Boehringer Mannheim) to the reaction solution, thereby amplifying and labeling each gene-specific fragment.

Using each of the thus amplified and labeled gene-specific fragments as a probe, hybridization and detection of positive plaques were carried out in accordance with the manual provided by Boehringer Mannheim.

DIG Nucleic Acid Detection Kit (manufactured by Boehringer Mannheim) was used for the detection.

### (B-1) Preparation of IgA nephropathy patient leukocyte cDNA. library

A 50 ml portion of blood sample was collected from each of four patients with IgA nephropathy, and each of the blood samples was centrifuged using Polymorphprep to isolate respective leukocyte fractions. The specific method was described in the manual attached to the Polymorphprep.

Using the thus isolated leukocytes, total RNAs were prepared by employing the guanidine thiocyanate-cesium trifluoroacetate method [Methods in Enzymology, 154, 3 (1987)]. From a total of 200 ml of blood samples, 320.7 µg of total RNAs was obtained.

A 272.6 µg portion of the thus obtained total RNAs was passed through an oligo(dT) cellulose column to obtain 10. 7 µg of mRNA as poly (A) ⁺ RNA.

In the same manner, 6.9 µg of mRNA was obtained from other four patients of IgA nephropathy.

Using 10.0 µg and 6.4 µg of the thus obtained respective mRNA samples, synthesis of cDNA, addition of *Eco*RI adapter and digestion reaction with *Xho*I were carried out using uniZAP-cDNA Synthesis Kit (manufactured by Stratagene), and the resulting fragments were inserted between *Eco*RI/*Xho*I of λ Zap II by ligation to prepare a cDNA library in which the cDNA was inserted in such a direction that its 5'-end was always present in the *Eco*RI site side of the vector.

The above specific method was described in the manual provided by Stratagene.

After packaging using a λ phage packaging kit Gigapack III Gold packaging extract (manufactured by Stratagene), *Escherichia coli* xL1-Blue MRF' was infected with the obtained library. The thus obtained cDNA library was used as the final cDNA library. The packaging and infection were carried out in accordance with the manual provided by Stratagene.

### (B-2) Preparation of neuroblastoma cell line NB-1 cDNA

Using RPMI 1640 medium (manufactured by Nissui Pharmaceutical) containing 10% fetal calf serum (manufactured by Biotech International), 2% penicillin (5,000 units/ml) streptomycin (5 mg/ml) solution (manufactured by Life Technology), 0.19% NaHCO₃ (manufactured by Sigma) and 4 mM glutamine, culturing and subculturing of a neuroblastoma cell line NB-1 (The Autonomic Nervous System, 10, 115 (1973), available from Human Science Research Resource Bank as JCRB0621) were carried out at 37°C in an atmosphere of 5% CO₂, and 1.25 × 10⁸ of confluent cells were recovered.

After washing of the thus recovered cells with PBS, 10.2 µg of purified mRNA was obtained using Fast Track mRNA Isolation Kit (manufactured by Invitrogen).

A 6 µg portion of the thus obtained mRNA and 1.5 µg of NotI-primer-adapter (manufactured by Promega) were put into a container, adjusted to 7 µl by adding distilled water, heated at 70°C for 10 minutes and then rapidly cooled on an ice bath.

The thus rapidly cooled solution was mixed with 4 µl of 5 × reverse transcriptase reaction buffer (attached to the enzyme), 2 µl of 100 mM DTT, 1 µl of 10 mM dNTP and 1 µl of [α-^{32p}] dCTP (110 TBq/mmol; manufactured by Amersham) as a tracer, and the mixture was incubated at 37°C for 2 minutes, mixed with 5 µl of (1,000 units) of a reverse transcriptase, SUPERSCRIPT II RNase H⁻ Reverse Transcriptase, and then allowed to react at 44°C for 1 hour to synthesize a cDNA.

The thus obtained reaction solution was mixed with 82 µl of distilled water, 32 µl of 5 × reaction buffer [100 mM Tris-HCl, 500 mM KCl, 25 mM MgCl₂, 50 mM (NH₄)₂SO₄, 10 mM DTT, 250 mg/ml bovine serum albumin (BSA), 750 mM β-nicotinamide dinucleotide] , 2.75 µl of 10 mM dNTP, 2.75 µl of [α-³²P] dCTP, 5.5 µl of 100 mM DTT, 2.5 µl of 6 units/µl *E*. *coli* DNA ligase (manufactured by Takara Shuzo), 11.5 µl of 3.5 units/µl *E. coli* DNA polymerase (manufactured by Takara Shuzo) and 2 µl of 0.6 unit/µl of E. coli ribonuclease H (manufactured by Takara Shuzo), and the thus prepared mixture was allowed to react at 16°C for 3 hours to decompose the mRNA and obtain a double-stranded cDNA.

The reaction solution was mixed with 4.8 µl of 1 unit/µl T4 DNA polymerase (manufactured by Takara Shuzo) and subjected to 5 minutes of the reaction at 16°C to form blunt ends at both termini.

The reaction solution was mixed with 2 µl of 500 mM EDTA (pH 8.0) and 2 µl of 10% sodium dodecyl sulfate (SDS) to terminate the reaction and then extracted with phenol-chloroform to denature and remove the enzyme. An aqueous layer was obtained.

In order to remove the cDNA of 400 bp or less in length and unreacted *Not*I-primer-adapter and nucleotide, the thus obtained aqueous layer was put on SizeSep-400 span column (manufactured by Pharmacia) which had been equilibrated with TE buffer and centrifuged at 400 g for 2 minutes, and the resulting eluate was subjected to ethanol precipitation to recover the cDNA.

The thus recovered cDNA was dissolved by adding 5 µl (50 pmol) of *Eco*RI adapter (manufactured by Promega) and mixed with 40 µl of the (A) solution and then with 5 µl of the (B) solution of Ligation Kit Ver.1 (manufactured by Takara Shuzo), and the resulting mixture was allowed at 15°C for 2 hours to effect addition of the *Eco*RI adapter to both termini of the cDNA.

The reaction solution was mixed with 40 µl of 10 mM EDTA (pH 8.0) and heated at 65°C for 15 minutes to terminate the reaction, and then the cDNA was recovered by ethanol precipitation.

The thus recovered cDNA was dissolved in 36 µl of distilled water and mixed with 5 µl of 10 × reaction buffer [500 mM Tris-HCl (pH 7.6), 100 mM MgCl₂] , 2.5 µl of 100 mM DTT, 2.5 µl of 10 mM ATP and 4 µl of 6 units/µl T4 polynucleotide kinase (manufactured by Takara Shuzo), and the mixture was allowed to react at 37°C to for 30 minutes to phosphorylate the 5'-end of the added *Eco*RI adapter.

The reaction solution was mixed with 7.2 µl of distilled water, 1.8 µl of 5 M NaCl and 8 units (1 µl) of NotI, and the mixture was subjected to 2 hours of the reaction at 37°C to cut off the *Not*I site in the NotI-primer-adapter.

After adding 6 µl of 500 mM EDTA to terminate the reaction, the reaction solution was mixed with 1 µl of 20 µg/µl tRNA and then extracted with phenol-chloroform to denature and remove the enzyme. An aqueous layer was obtained.

In order to remove unreacted *Eco*RI adapter, the thus obtained aqueous layer was put on SizeSep-400 span column which had been equilibrated with TE buffer and centrifuged at 400 g for 2 minutes to recover the eluate.

The thus recovered eluate was overlaid on potassium acetate solution having a concentration gradient of from 5 to 20%, ultracentrifuged at 50,000 rpm for 3 hours. Then, 21 fractions were recovered from the bottom of the centrifugation tube using a peristaltic pump.

Each of the fractions was subjected to ethanol precipitation to recover cDNA, a portion of each of the thus recovered samples was subjected to agarose gel electrophoresis and then to autoradiography to measure the length of cDNA contained in each fraction, and the samples were recovered in three fractions, namely a fraction (H) containing cDNA of about 3 kb or more, a fraction (M) containing cDNA of 1 to 3 kb and a fraction (L) containing cDNA of 1 kb or less.

A 9 µg (9 µl) portion of a cloning vector ZAP II (manufactured by Stratagene) was mixed with 10 µl of 10 x H restriction enzyme buffer (manufactured by Takara Shuzo) , 75 µl of distilled water and 90 units (6 µl) of *Eco*RI, and the mixture was subjected to 2 hours of the reaction at 37°C.

The reaction solution was mixed with 1 µl of 5 M NaCl and 40 units (5 µl) of *Not*I, allowed to react at 37°C for 2 hours, and further mixed with 8 units (1 µl) of NotI and again subjected to 1 hour of the reaction at 37C° to cleave the *Eco*RI site and NotI site of the vector.

The reaction solution was mixed with 100 µl of 2 M Tris-HCl (pH 8.0) and 1 unit (2 µl) of *E*. *coli* C75 alkaline phosphatase (manufactured by Takara Shuzo) and allowed to react at 60°C for 30 minutes to dephosphorylate the 5'-ends of the vector cleaved by *Eco*RI and NotI, and then these enzymes were removed by repeating phenol-chloroform extraction twice.

After removal of the enzymes, chloroform extraction was carried out and the resulting water layer was subjected to ethanol precipitation to recover the vector DNA which was subsequently dissolved in TE buffer.

Each of the cDNA samples recovered in three fractions was mixed with 1 µg of the vector DNA and subjected to ethanol precipitation, and the thus recovered vector DNA and cDNA were dissolved in 4 µl of a ligase buffer [100 mM Tris-HCl (pH 7.6), 5 mM MgCl₂ , 300 mM NaCl], mixed with 4 µl of the (B) solution of Ligation Kit Ver.1 and then allowed to react at 26°C for 10 minutes to ligate the cDNA to the vector DNA.

A 4 µl portion of each of the reaction solutions was subjected to packaging using a λ phage packaging kit, GigaPack Gold II (manufactured by Stratagene). The reagents and methods were described in the manual attached to the kit.

*E. coli* XL1-Blue MRF' was infected with the thus obtained phage and the titer was measured. Thereafter, the cDNA library was amplified once by growing the phage on a plate medium and recovering it in SM buffer and used as the final cDNA library. The measurement of titer and amplification of library were carried out in accordance with the manual attached to the λ phage packaging kit. A library prepared from the (H) fraction containing cDNA of about 3 kb or more was used for the screening of the present invention.

### (C) 5'-RACE

5'-RACE of the IgA nephropathy patient cDNA prepared in the above method (B) was carried out using 5'-RACE System ver.2 (manufactured by Life Technologies). The specific method was described in the manual attached to the kit.

Using the above methods (A) to (C), cDNA cloning of the seven genes shown in Table 2 was achieved.

**TABLE 2**

| Gene name | SEQ ID NO. | cDNA clone | Method¹⁾ | cDNA source |
|---|---|---|---|---|
| INP303A | 1 | GTINP303A-41a | A | human leukocytes |
| | | INP303A ph1-3 | B | NB-1 |
| | | INP303A-R1 | C | IgA nephropathy leukocytes |

| | | | | |
|---|---|---|---|---|
| 1) Cloning method of each cDNA clone obtained: A: gene trapper method, B: plaque hybridization of cDNA library C: 5' -RACE method. | | | | |

Nucleotide sequence of the cDNA moiety of each of the thus obtained cDNA clones was determined using 377 DNA Sequencer manufactured by Perkin Elmer. Determination of the nucleotide sequence was carried out using Dye cycle sequencing FS Ready Reaction Kit in accordance with the manual attached to the kit. Additionally, the nucleotide sequence was translated into amino acid sequence by three frames to examine whether an open reading frame (ORF) composed of 100 or more amino acids is present.

### (1) INP303A

A cDNA clone GTINP303A-41a was obtained by the gene trapper method, but this was considered to be an incomplete cDNA clone because of the absence of ORF, which corresponds to 100 or more amino acids, in the nucleotide sequence of the cDNA.

In order to obtain a full length cDNA clone, 5' -RACE was carried out using specific primers (nucleotide sequences are shown in SEQ ID NO:110 and NO:111) which correspond to a moiety close to the 5'-end of GTINP303A-41a to obtain cDNA clone INP303A-R1. Also, since a part of the cDNA nucleotide sequence of GTINP303A-41a was not able to determine, another cDNA clone INP303A-ph1-3 was obtained from an NB-1 cDNA library by plaque hybridization.

By combining nucleotide sequences of these cDNA clones thus obtained, a 4,276 bp nucleotide sequence of the cDNA of INP303A was determined as shown in SEQ ID NO:1.

The nucleotide sequence of a fragment obtained by differential display (SEQ ID NO:41) coincided with the complementary chain nucleotide sequence corresponding to the positions 2,797 to 3,101 of SEQ ID NO:1. Therefore, it was considered that the anchor primer was not annealed to the 3'-end poly(A) sequence of mRNA but to the complementary strand of the sequence wherein continuous T's were present at the 2782nd to 2795th positions in SEQ ID NO:1.

An ORF corresponding to 239 amino acids (corresponds to the positions 53 to 742 of SEQ ID NO:1, the amino acid sequence is shown in SEQ ID NO:34) was found in the nucleotide sequence of the cDNA of INP303A-R1.

When the amino acid sequence of the ORF was compared with an amino acid data base, it was found that this sequence has a homology with C4OH1 which was estimated to be a protein encoded by a Nematoda genomic gene clone C4OH1, mouse cytoplasmic polyadenylation element binding protein (CPEBP) and Drosophila orb gene.

It was found also that an amino acid sequence just downstream of the region where these proteins showed a homology with the INP303A protein also showed a homology with the amino acid sequence encoded by the nucleotide sequence of positions 3,346 to 3,577 of SEQ ID NO:1. Therefore, it was assumed that this cDNA is a result of abnormal splicing in which a 2,689 bp nucleotide sequence (corresponds to positions 713 to 3,352 in SEQ ID NO:1) which seems to be an intron originally remained in the nucleotide sequence of INP303A.

It was found that the nucleotide sequence of a fragment which was obtained by the differential display and whose expression quantity increased in IgA nephropathy patients is present in this insertion sequence and the amount of mRNA which caused such an abnormal splicing increases in IgA nephropathy patients. It is highly possible that a protein translated from an mRNA which caused the abnormal splicing does not exert its original function, because its amino acid sequence at and after the 220 position is different from the original protein encoded by INP303A, namely a protein (295 amino acids) encoded by a nucleotide sequence resulting from the elimination of intron deduced from the a homology.

### Example 4: Production of polyclonal antibody against the protein of the present invention

### (1) Preparation of antigen

The amino acid sequence of each protein was analyzed and parts which were considered to be suitable for peptide antigens were selected from the amino acid sequences of highly hydrophilic parts, the N-end and the C-end, and Compounds 1 to 4 (SEQ ID NO:114 to NO: 117) were synthesized. Physicochemical properties of these compounds were measured by the following methods.

Mass spectrometry was carried out according to the FAB-MS method using JMS-HX110A (manufactured by Japan Electron Optics Laboratory). Amino acids were analyzed according to the method of Cohen, S.A. et al. [Analytical Biochemistry, 222, 19 (1994)]. Hydrolysis was carried out in hydrochloric acid vapor at 110°C for 20 hours and the amino acid composition of a hydrolysate was analyzed using an amino acid analyzer, Waters AccQ-Tag (manufactured by Waters).

### (i) Synthesis of peptide having the amino acid sequence represented by SEQ ID NO:114 (Compound 1) (Comparative Example)

To produce an antibody against the proteins of SEQ ID NO:37 and NO:38, a peptide having the amino acid sequence corresponding to the C-terminal 14 amino acids of these proteins was synthesized according to the following method.

In a reactor of an automatic synthesizer (manufactured by ACT), 52.1 mg of a carrier resin to which 25 µmol of N^{α}-9-fluorenylmethyloxycarbonyl-L-isoleucine (hereinafter referred to as "Fmoc-Ile") had been bound (Wang Resin, manufactured by Novabiochem) was introduced, and 0.5 ml of DMF was added thereto, followed by stirring for 3 minutes. Then the solution was discarded and the following operations were carried out.
(a) To the mixture, 1 ml of a solution (hereinafter referred to as the "25% piperidine-DMF solution") obtained by 25% of piperidine was added to N,N-dimethylformamide (hereinafter referred to as "DMF") was added, followed by stirring for 2 minutes, and the solution was discarded. To the mixture, 1 ml of the 25% - piperidine-DMF solution was added again, followed by stirring for 10 minutes, and the solution was discarded.
(b) To the carrier resin, 1 ml of DMF was added. After stirring for 1 minute, the solution was discarded. After repeating this operation 6 times, the resin and the reactor were washed with 0.5 ml of DMF.
   Thus, a carrier resin from which 9-fluorenylmethyloxycarbonyl (hereinafter referred to as "Fmoc") had been removed and to which H-Ile had been bound was obtained.
(c) To the resulting carrier resin, 500 µl of DMF, 250 µl of an N-methylpyrrolidone (hereinafter referred to as "NMP") solution containing 0.5 M of N^{α}-9-fluorenylmethyloxycarbonyl-L-aspartic acid-β-t-butyl ester [hereinafter referred to as "Fmoc-Asp(OtBu)-OH"] and 0.5 M of N-hydroxybenzotriazole (hereinafter referred to as "HOBt") monohydrate, and 125 µl of an NMP solution containing 0.5 M of N,N'-diisopropylcarbodiimide (hereinafter referred to "as DIPC") were added, followed by stirring for 10 minutes and allowing to stand for 1 minute. After repeating the cycle of stirring for 10 minutes and allowing to stand for 1 minute 4 times, the solution was discarded.
(d) To the carrier resin, 1 ml of DMF was added, followed by stirring for 1 minute, and the solution was discarded. This operation was repeated twice.
(e) To the carrier resin, 375 µl of DMF, 250 µl of an NMP solution containing 0.5 M of Fmoc-Asp(OtBu)-OH and 0.5 M of HOBt monohydrate, 250 µl of a DMF solution containing 0.5 M of 2-(1H-benzotriazol-1-yl)-1,1,3,3-tetramethyluronium hexafluorophosphate (hereinafter referred to as "HBTU"), and 125 µl of a DMF solution containing 2 M of diisopropylethylamine (hereinafter referred to as "DIEA") were added, followed by stirring for 10 minutes and allowed to stand for 1 minute. After repeating the cycle of stirring for 10 minutes and allowing to stand for 1 minute three times, the solution was discarded.
(f) To the carrier resin, 1 ml of DMF was added, followed by stirring for 1 minute, and the solution was discarded. After repeating this operation twice, the resin and the reactor were washed with 0.5 ml of DMF.

Thus, a carrier to which Fmoc-Asp(OtBu)-Ile had been bound was obtained.

After completion of the above-described step (f), the operations of (a) and (b) for removing the Fmoc groups were carried out. After removing the Fmoc groups, a condensation reaction was carried out as in the step (c) but replacing Fmoc-Asp(OtBu)-OH with N^{α}-9-fluorenylmethyloxycarbonyl-O-t-butyl-L-threonine [hereinafter referred to as "Fmoc-Thr(tBu)-OH"]. After completion of the condensation reaction, the washing operation of (d) was carried out, and then a condensation reaction was carried out again as in the step (e) but replacing Fmoc-Asp(OtBu)-OH with Fmoc-Thr(tBu)-OH, followed by the washing operation of the step (f). Thus, Fmoc-Thr(tBu)-Aps(OtBu)-Ile was synthesized on the carrier.

The operations in the steps (a) to (f) were repeated by successively using, in the steps (c) and (e), N^{α}-9-fluorenylmethyloxycarbonyl-N⁹-2,2,5,7,8-pentamethylchroman-6-sulfonyl-L-arginine [hereinafter referred to as "Fmoc-Arg(Pmc)-OH"], Fmoc-Thr(tBu)-OH, N^{α}-9-uorenylmethyloxycarbonyl-L-phenylalanine (hereinafter referred to as "Fmoc-Phe-OH"), Fmoc-Phe-OH, Fmoc-Thr(tBu)-OH, N^{α}-9-fluorenylmethyloxycarbonyl-L-glycine (hereinafter referred to as "Fmoc-Gly-OH"), Fmoc-Asp(OtBu)-OH, N^{α}-9-fluorenylmethyloxycarbonyl-L-glutamic acid-γ-t-butyl ester [hereinafter referred to as "Fmoc-Glu(Ot-Bu)-OH"], Fmoc-Arg(Pmc)-OH, N^{α}-9-fluorenylmethyloxycarbonyl-N^{ε}-t-butyloxycarbonyl-L-lysine [hereinafter referred to "as Fmoc-Lys(Boc)-OH"], Fmoc-Thr(tBu)-OH, N^{α}-9-fluorenylmethyloxycarbonyl-L-leucine (hereinafter referred to as "Fmoc-Leu-OH"), Fmoc-Thr(tBu)-OH, N^{α}-9-fluorenylmethyloxycarbonyl-L-proline (hereinafter referred to as "Fmoc-Pro-OH"), and N^{α}-9-fluorenylmethyloxycarbonyl-S-trityl-L-cysteine [hereinafter referred to as "Fmoc-Cys(Trt)-OH"), and then the operations of the steps (a) and (b) for removing the Fmoc groups were carried out.

The resin thus obtained was washed successively with methanol and butyl ether and dried under reduced pressure for 12 hours to give a carrier resin to which a side chain-protected peptide had been bound. To the carrier resin, 1 ml of a mixture solution composed of 82.5% of trifluoroacetic acid (hereinafter referred to as "TFA"), 5% of thioanisole, 5% of water, 3% of ethyl methyl sulfide, 2.5% of 1,2-ethanediol and 2% of thiophenol was added, followed by allowing to stand at room temperature for 8 hours, to remove the side chain-protective group and cut out the peptide from the resin. After filtering off the resin, about 10 ml of ether was added to the resulting solution. The precipitate thus formed was collected by centrifugation and decantation to give 49.0 mg of a crude peptide. This crude product was dissolved in a 2M aqueous acetic acid solution and then purified by HPLC using a reversed phase column (CAPCELL PAK C18, 30 mm I.D. × 250 mm, manufactured by Shiseido). Then it was eluted by the linear concentration gradient method, wherein a 90% aqueous acetonitrile solution containing 0.1% of TFA was added to a 0.1% aqueous TFA solution, followed by detection at 220 nm to give a fraction containing Compound 1. This fraction was freeze-dried to give 18.0 mg of Compound 1.
Mass spectrometry [FABMS]; m/z = 1797.4 (M+H⁺)
Amino acid analysis: Asx 2.1(2), Glx 1.1(1), Gly 1.0(1), Arg 1.8 (2), Thr 2.0(2), Pro 0.9 (1) , Cys 1.3 (1) , Lys 1.0(1), Ile 1.0(1), Leu 1.0(1), Phe 1.9(2)

### (iv) Synthesis of peptide having the amino acid sequence represented by SEQ ID NO:117 (Compound 4)

To produce an antibody against the protein of SEQ ID NO: 34, a peptide having the amino acid sequence corresponding to the C-terminal 15 amino acids of this protein was synthesized according to the following method.

As a starting material, 49.0 mg of a carrier resin to which 25 µmol of Fmoc-Phe was bound (Wang Resin, manufactured by Novabiochem) was used, and condensation was successively carried out using Fmoc-Lys(Boc)-OH, Fmoc-Gly-OH, Fmoc-Phe-OH, Fmoc-His(Trt)-OH, Fmoc-Leu-OH, Fmoc-Ile-OH, Fmoc-Leu-OH, Fmoc-Ser (tBu) -OH, Fmoc-Val-OH, Fmoc-Arg(Pmc)-OH, Fmoc-Lys(Boc)-OH, Fmoc-Asp (OtBu) -OH, Fmoc-Ile-OH, Fmoc-Glu(OtBu)-OH and Fmoc-Cys(Trt)-OH in the same manner as in (1) - (i) . Removing of the Fmoc groups, washing and drying were carried out to give a carrier resin to which a side chain-protected peptide had been bound. The side chain-protective group was cleaved and the peptide was cut out from the resin in the same manner as in (1) - (i) to give 41.2 mg of a crude peptide. It was purified by HPLC with the use of a reversed phase column, 14.6 mg of Compound 4 was obtained.
Mass spectrometry [FABMS); m/z = 1905.8 (M+H⁺)
Amino acid analysis: Asx 1.0(1), Ser 0.9(1), Glx 1.0(1), Gly 1.1 (1) , His 1.1 (1) , Arg 0.9 (1) , Cys 1.3 (1), Val 1.0 (1) , Lys 2.1 (2) , Ile 1.8 (2), Leu 1.9 (2), Phe 2.2 (2)

### (2) Preparation of immunogen

To enhance immunogenicity, conjugates of Compounds 1 to 8 obtained in the above (1) with KLH (manufactured by Calbiochem) were prepared in the following manner and employed as antigens.

Namely, KLH was dissolved in PBS to give a concentration of 10 mg/ml, and 25 mg/ml of MBS (manufactured by Nakalai Tesque) at 1/10 volume was added dropwise thereto, followed by reaction under stirring for 30 minutes.

The unreacted MBS was removed using Sephadex G-25 column which had been preliminarily equilibrated with PBS.

To 0.1 M sodium phosphate buffer (pH 7.0) in which 1 mg of each of Compounds 1 to 8 had been dissolved, 2.5 mg of the resulting KLH-MB was added and mixed, followed by reaction under stirring at room temperature for 3 hours.

After completion of the reaction, the reaction solution was dialyzed against PBS.

### (3) Immunization of animals and preparation of polyclonal antibodies

To female (SD) rats of 5 weeks of age, 100 µg of the conjugate of each compound with KLH as prepared in the above (2) was administered, together with 2 mg of an aluminum gel and 1×1.0⁹ pertussis vaccine cells (manufactured by Chiba Serum Institute) . Two weeks after the administration, 100 µg of the conjugate was administered once a week 4 times in total. The blood of each animal was collected from the venous plexus of the eyeground and the serum antibody titer was examined by the enzyme immunoassay. The whole blood was collected from rats showing an enough titer to obtain the serum.

### (4) Enzyme immunoassay

As the antigen for the assay, conjugates of respective compounds obtained in the above (1) with thyroglobulin (hereinafter referred to as "THY") were used. The production was carried out according to the above (2), except that MBS was replaced with SMCC (manufactured by Sigma) as a crosslinking agent. Into a 96-well EIA plate (manufactured by Greiner), 10 µg/ml of each conjugate thus prepared was poured at 50 µl/well and allowed to be stand for adsorption at 4°C overnight. After washing, 1% BSA-PBS was added at 100 µl/well and reaction was carried out at room temperature for 1 hour to block the remaining active groups. The 1% BSA-PBS was discarded, and the immunized rat antiserum was poured at 50 µl/well, followed by reaction for 2 hours. After washing with a solution of PBS to which 0.05% Tween had been added (hereinafter referred to as "0.05% Tween-PBS"), peroxidase-labeled rabbit anti-rat immunoglobulin (manufactured by Dako) was added at 50 µl/well, followed by reaction at room temperature for 1 hour. After washing with 0.05% Tween-PBS, color development was performed using an ABTS substrate solution [ammonium 2,2-adinobis(3-ethylbenzothiazole-6-sulfonate)], and the absorbance (OD) at 415 nm was measured with a plate reader (E-max, manufactured by Wako Pure Chemical Industries).

The results are shown in Figs. 1 to 8. Each of these antisera showed a specific reactivity to the compound used as the immunogen.

The diagnosis of IgA nephropathy can be effected using the DNA, protein and antibody of the present invention.

### FREE TEXT OF SEQUENCE LISTINGS

SEQ ID NO:47-Explanation of artificial sequence: Synthetic DNA
SEQ ID NO:48-Explanation of artificial sequence: Synthetic DNA
SEQ ID NO:107-Explanation of artificial sequence: Synthetic DNA
SEQ ID NO:114-Explanation of artificial sequence: Synthetic peptide
SEQ ID NO:115-Amidation, glutaminamide
SEQ ID NO:116-Amidation, glycinamide
SEQ ID NO:117-Explanation of artificial sequence: Synthetic peptide

## Claims

1. A DNA comprising a nucleotide sequence selected from the group consisting of :
(a) a DNA having the nucleotide sequence of SEQ ID NO:1;
(b) a DNA encoding a protein comprising the amino acid sequence of SEQ ID NO:34.

2. A vector comprising the DNA of claim 1.

3. A non-human transformant comprising the vector of claim 2.

4. A method for producing a protein encoded by the DNA of claim 1 comprising:
culturing the transformant of claim 3 in a medium to produce and accumulate said protein in the culture; and recovering said protein from the resulting culture.

5. A protein encoded by the DNA of claim 1 or obtainable by the method of claim 4.

6. An antibody which specifically recognizes a protein consisting of the amino acid sequence of SEQ ID NO:34.

7. A method for detecting mRNA of an IgA nephropathy-related gene using the DNA of claim 1.

8. A method for immunologically detecting the protein of claim 5 using the antibody of claim 6.

9. A diagnostic composition comprising the DNA of claim 1, the vector of claim 2, the protein of claim 5 or the antibody of claim 6 and, optionally, a diagnostically acceptable carrier.

10. Use of the DNA of claim 1, the vector of claim 2, the protein of claim 5 or the antibody of claim 6 for the preparation of a diagnostic composition for diagnosing IgA nephropathy.

## Patentansprüche

1. DNA umfassend eine Nucleotidsequenz ausgewählt aus der Gruppe von:
(a) einer DNA mit der Nucleotidsequenz von SEQ ID NO:1;
(b) einer DNA, die ein Protein codiert, das die Aminosäuresequenz von SEQ ID NO:34 umfasst.

2. Vektor, der die DNA nach Anspruch 1 umfasst.

3. Nicht-menschliche Transformante, die den Vektor nach Anspruch 2 umfasst.

4. Verfahren zur Produktion eines Proteins, das durch die DNA nach Anspruch 1 codiert wird, umfassend: Züchten der Transformante nach Anspruch 3 in einem Medium, um das Protein in der Kultur zu produzieren und anzureichern; und Gewinnen des Proteins aus der resultierenden Kultur.

5. Protein, das von der DNA nach Anspruch 1 codiert wird oder erhältlich ist durch das Verfahren nach Anspruch 4.

6. Antikörper, der spezifisch ein Protein bestehend aus der Aminosäuresequenz von SEQ ID NO:34 erkennt.

7. Verfahren zum Nachweis von mRNA eines mit IgA-Nephropathie in Beziehung stehenden Gens unter Verwendung der DNA nach Anspruch 1.

8. Verfahren zum immunologischen Nachweis des Proteins nach Anspruch 5 unter Verwendung des Antikörpers nach Anspruch 6.

9. Diagnostisches Mittel umfassend die DNA nach Anspruch 1, den Vektor nach Anspruch 2, das Protein nach Anspruch 5 oder den Antikörper nach Anspruch 6 und gegebenenfalls einen diagnostisch verträglichen Träger.

10. Verwendung der DNA nach Anspruch 1, des Vektors nach Anspruch 2, des Proteins nach Anspruch 5 oder des Antikörpers nach Anspruch 6 für die Herstellung eines diagnostischen Mittels zur Diagnose von IgA-Nephropathie.

## Revendications

1. ADN comprenant une séquence nucléotidique choisie parmi le groupe consistant en :
(a) un ADN ayant la séquence nucléotidique de SEQ ID NO: 1;
(b) un ADN codant pour une protéine comprenant la séquence d'aminoacides de SEQ ID NO :34.

2. Vecteur comprenant l'ADN selon la revendication 1.

3. Transformant non humain comprenant le vecteur selon la revendication 2.

4. Procédé pour produire une protéine codée par l'ADN selon la revendication 1 consistant à :
mettre en culture le transformant selon la revendication 3 dans un milieu pour produire et accumuler ladite protéine dans la culture ; et recueillir ladite protéine à partir de la culture résultante.

5. Protéine codée par l'ADN selon la revendication 1 ou qui peut être obtenue par le procédé selon la revendication 4.

6. Anticorps qui reconnaît spécifiquement une protéine consistant en la séquence d'aminoacides de SEQ ID NO: 34.

7. Procédé pour détecter l'ARNm d'un gène associé à une néphropathie de l'IgA utilisant l'ADN selon la revendication 1.

8. Procédé pour détecter de manière immunologique la protéine selon la revendication 5 en utilisant l'anticorps selon la revendication 6.

9. Composition diagnostique comprenant l'ADN selon la revendication 1, le vecteur selon la revendication 2, la protéine selon la revendication 5 ou l'anticorps selon la revendication 6 et, éventuellement, un support acceptable de manière diagnostique.

10. Utilisation de l'ADN selon la revendication 1, du vecteur selon la revendication 2, de la protéine selon la revendication 5 ou de l'anticorps selon la revendication 6 pour la préparation d'une composition diagnostique pour diagnostiquer une néphropathie de l'IgA.
